# EUROPEAN PATENT APPLICATION

(11) **EP 4 059 954 A1**
(43) Date of publication of application: **21.09.2022**
(21) Application number: 20886905.7
(22) Date of filing: 12.11.2020
(51) Int. Cl.: C07K 14/47, C07K 14/81, C12N 15/12, C12N 15/15, C12Q 1/02, G01N 33/574

(54) **METHOD, KIT, AND BIOMARKER FOR ASSISTING IN DIAGNOSIS OF COLON CANCER**

(30) Priority: 15.11.2019 JP 2019206727
(71) Applicant: FUJIFILM Corporation, Tokyo 106-8620 (JP); Public University Corporation Yokohama City University, Yokohama-shi, Kanagawa 236-0027 (JP)
(72) Inventor: KAWASAKI, Nana, Yokohama-shi, Kanagawa 230-0045 (JP); OHTA, Yuki, Yokohama-shi, Kanagawa 230-0045 (JP)
(74) Representative: Klunker IP Patentanwälte PartG mbB
(86) International application number: PCT/JP2020/042344
(87) International publication number: WO 2021/095824

(57) **Abstract**

An object of the present invention is to provide a biomarker capable of diagnosing colon cancer.

The present invention relates to the biomarker for assisting diagnosis of colon cancer, including a sugar chain selected from the following sugar chains (1) to (4),
(1) at least one sugar chain represented by the following formula 1 or 2 which is present in alpha 1-antitrypsin;
(2) a sugar chain represented by the following formula 1 which is present in leucine-rich alpha 2-glycoprotein;
(3) at least one sugar chain represented by the following formula 1 or 2 which is present in alpha 1-antichymotrypsin; and
(4) a sugar chain represented by the following formula 1 which is present in a complement component 9.

SEQUENCE LISTING

<110>  FUJIFILM Wako Pure Chemical Corporation
       Public University Corporation Yokohama City University

<120>  Method for assisting the diagnosis of colon cancer, kit and biomarker

<130>  2139PCT

<150>  JP2019-206727
<151>  2019-11-15

<160>  6

<170>  PatentIn version 3.5

<210>  1
<211>  30
<212>  PRT
<213>  Homo sapiens

<400>  1

Gln Leu Ala His Gln Ser Asn Ser Thr Asn Ile Phe Phe Ser Pro Val
1               5                   10                  15

Ser Ile Ala Thr Ala Phe Ala Met Leu Ser Leu Gly Thr Lys
            20                  25                  30

<210>  2
<211>  46
<212>  PRT
<213>  Homo sapiens

<400>  2

Ser Asp His Gly Ser Ser Ile Ser Cys Gln Pro Pro Ala Glu Ile Pro
1               5                   10                  15

Gly Tyr Leu Pro Ala Asp Thr Val His Leu Ala Val Glu Phe Phe Asn
            20                  25                  30

Leu Thr His Leu Pro Ala Asn Leu Leu Gln Gly Ala Ser Lys
        35                  40                  45

<210>  3
<211>  13
<212>  PRT
<213>  Homo sapiens

<400>  3

Leu Pro Pro Gly Leu Leu Ala Asn Phe Thr Leu Leu Arg
1               5                   10

<210>  4
<211>  31

<212>  PRT
<213>  Homo sapiens

<400>  4

Gln Leu Asp Met Leu Asp Leu Ser Asn Asn Ser Leu Ala Ser Val Pro
1               5                   10                  15

Glu Gly Leu Trp Ala Ser Leu Gly Gln Pro Asn Trp Asp Met Arg
            20                  25                  30

<210>  5
<211>  30
<212>  PRT
<213>  Homo sapiens

<400>  5

Asn Val Ile Phe Ser Pro Leu Ser Ile Ser Thr Ala Leu Ala Phe Leu
1               5                   10                  15

Ser Leu Gly Ala His Asn Thr Thr Leu Thr Glu Ile Leu Lys
            20                  25                  30

<210>  6
<211>  18
<212>  PRT
<213>  Homo sapiens

<400>  6

Ala Val Asn Ile Thr Ser Glu Asn Leu Ile Asp Asp Val Val Ser Leu
1               5                   10                  15

Ile Arg

## Description

### Technical Field

The present invention relates to a method, a kit, and a biomarker for assisting diagnosis of colon cancer.

### Background Art

A sugar chain is a general term for molecules in which monosaccharides such as glucose, galactose, mannose, fucose, xylose, N-acetylglucosamine, and N-acetylgalactosamine, and derivatives thereof are bound in a chain via glucoside bonds.

Sugar chains are mainly present on the surface of cells in the form of complex carbohydrates bound to proteins and lipids in a living body and are involved in physiological actions such as cell proliferation, infection with bacteria and viruses, nerve elongation, inflammation, and immunity.

Meanwhile, it is also known that the structure of sugar chains changes with diseases such as colon cancer. In recent years, research for using sugar chains as biomarkers for diseases such as colon cancer have been actively conducted.

As the biomarkers, for example, a carcinoembryonic antigen (CEA), a leucine-rich alpha 2-glycoprotein, and the like are known (JP2005-184168A).

### Citation List

### Patent Literature

Patent Literature 1: JP2015-184168A

### Summary of Invention

### Technical Problem

However, the known biomarkers are not sufficient in terms of accuracy.

An object of the present invention is to provide a new biomarker, a new kit, and a new method of assisting diagnosis of colon cancer.

Particularly, another object of the present invention is to provide a biomarker for colon cancer with high accuracy.

### Solution to Problem

The present invention has been made for the purpose of solving the problems, and has the following configurations.
[1] A biomarker for assisting diagnosis of colon cancer, comprising: a sugar chain selected from the following sugar chains (1) to (4),
   (1) at least one sugar chain represented by the following formula 1 or 2 which is present in alpha 1-antitrypsin;
   (2) a sugar chain represented by the following formula 1 which is present in leucine-rich alpha 2-glycoprotein;
   (3) at least one sugar chain represented by the following formula 1 or 2 which is present in alpha 1-antichymotrypsin; and
   (4) a sugar chain represented by the following formula 1 which is present in a complement component 9, 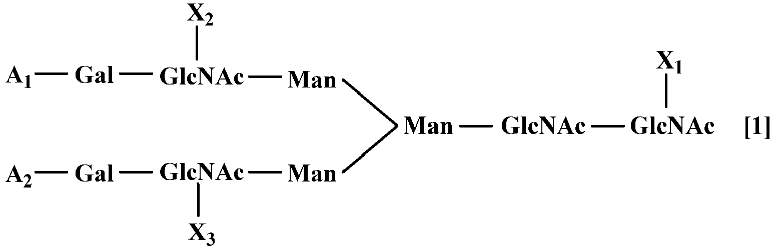
      (in the formula 1, X₁, X₂, and X₃ each independently represent Fuc or H, and A₁ and A₂ each independently represent Neu5Ac, Neu5Ac-Neu5Ac, or H) 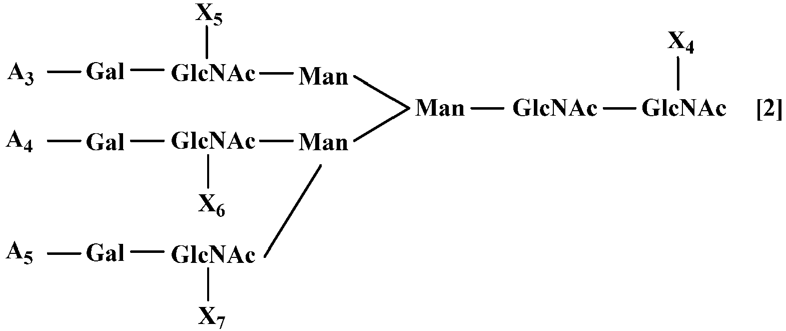
      (in the formula 2, X₄, X₅, X₆, and X₇ each independently represent Fuc or H, and A₃, A₄, and A₅ each independently represent Neu5Ac, Neu5Ac-Neu5Ac, or H).
[2] The biomarker according to [1], in which the sugar chain is a sugar chain represented by any one of the following sugar chains (1-1) to (4-1),
   (1-1) at least one sugar chain represented by the formula 1 or 2 which is bound to an asparagine residue at position 70 from a N-terminal of an amino acid sequence of the alpha 1-antitrypsin;
   (2-1) a sugar chain represented by the formula 1 which is bound to an asparagine residue at position 79, an asparagine residue at position 186, or an asparagine residue at position 269 from a N-terminal of an amino acid sequence of the leucine-rich alpha 2-glycoprotein;
   (3-1) at least one sugar chain represented by the formula 1 or 2 which is bound to an asparagine residue at position 93 from a N-terminal of an amino acid sequence of the alpha 1-antichymotrypsin; and
   (4-1) a sugar chain represented by the formula 1 which is bound to an asparagine residue at position 415 from a N-terminal of an amino acid sequence of the complement component 9.
[3] The biomarker according to [1] or [2], in which the sugar chain is at least one sugar chain represented by the formula 1 or 2 which is bound to an asparagine residue at position 70 from a N-terminal of an amino acid sequence of the alpha 1-antitrypsin.
[4] A method of assisting diagnosis of colon cancer, comprising: measuring a sugar chain selected from the following sugar chains (1) to (4) in a sample derived from a test animal; and determining whether the test animal has colon cancer based on an obtained measurement result,
   (1) at least one sugar chain represented by the following formula 1 or 2 which is present in alpha 1-antitrypsin;
   (2) a sugar chain represented by the following formula 1 which is present in leucine-rich alpha 2-glycoprotein;
   (3) at least one sugar chain represented by the following formula 1 or 2 which is present in alpha 1-antichymotrypsin; and
   (4) a sugar chain represented by the following formula 1 which is present in a complement component 9, 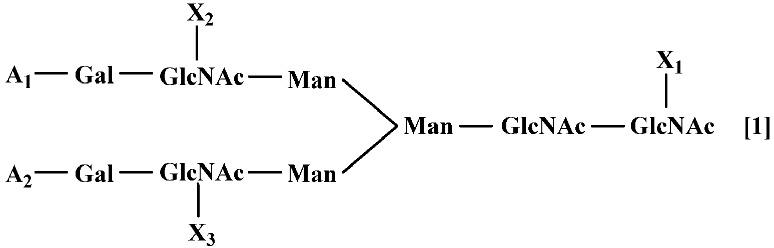
      (in the formula 1, X₁, X₂, and X₃ each independently represent Fuc or H, and A₁ and A₂ each independently represent Neu5Ac, Neu5Ac-Neu5Ac, or H) 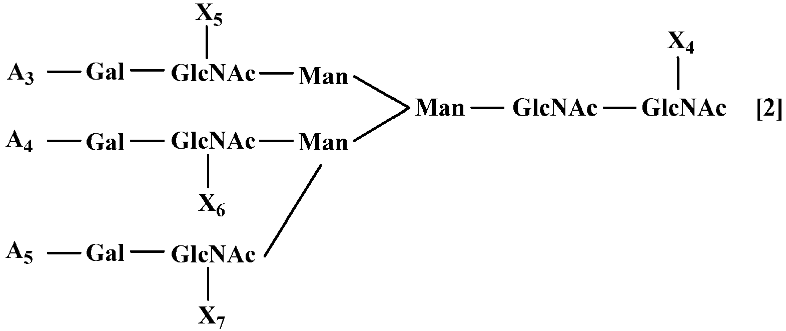
      (in the formula 2, X₄, X₅, X₆, and X₇ each independently represent Fuc or H, and A₃, A₄, and A₅ each independently represent Neu5Ac, Neu5Ac-Neu5Ac, or H).
[5] The method according to [4], in which the sugar chain is a sugar chain represented by any one of the following sugar chains (1-1) to (4-1),
   (1-1) at least one sugar chain represented by the formula 1 or 2 which is bound to an asparagine residue at position 70 from a N-terminal of an amino acid sequence of the alpha 1-antitrypsin;
   (2-1) a sugar chain represented by the formula 1 which is bound to an asparagine residue at position 79, an asparagine residue at position 186, or an asparagine residue at position 269 from a N-terminal of an amino acid sequence of the leucine-rich alpha 2-glycoprotein;
   (3-1) at least one sugar chain represented by the formula 1 or 2 which is bound to an asparagine residue at position 93 from a N-terminal of an amino acid sequence of the alpha 1-antichymotrypsin; and
   (4-1) a sugar chain represented by the formula 1 which is bound to an asparagine residue at position 415 from a N-terminal of an amino acid sequence of the complement component 9.
[6] The method according to [4] or [5], in which the sugar chain is at least one sugar chain represented by the formula 1 or 2 which is bound to an asparagine residue at position 70 from a N-terminal of an amino acid sequence of the alpha 1-antitrypsin.
[7] The method according to any one of [4] to [6], in which the sample is whole blood, serum, or plasma.
[8] A reagent kit for assisting diagnosis of colon cancer, comprising: a substance having an affinity for a sugar chain selected from the following sugar chains (1) to (4),
   (1) at least one sugar chain represented by the following formula 1 or 2 which is present in alpha 1-antitrypsin;
   (2) a sugar chain represented by the following formula 1 which is present in leucine-rich alpha 2-glycoprotein;
   (3) at least one sugar chain represented by the following formula 1 or 2 which is present in alpha 1-antichymotrypsin; and
   (4) a sugar chain represented by the following formula 1 which is present in a complement component 9, 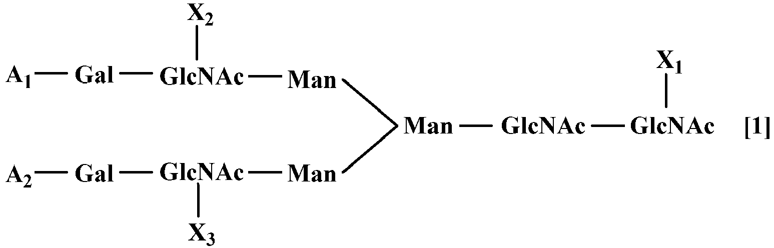
      (in the formula 1, X₁, X₂, and X₃ each independently represent Fuc or H, and A₁ and A₂ each independently represent Neu5Ac, Neu5Ac-Neu5Ac, or H) 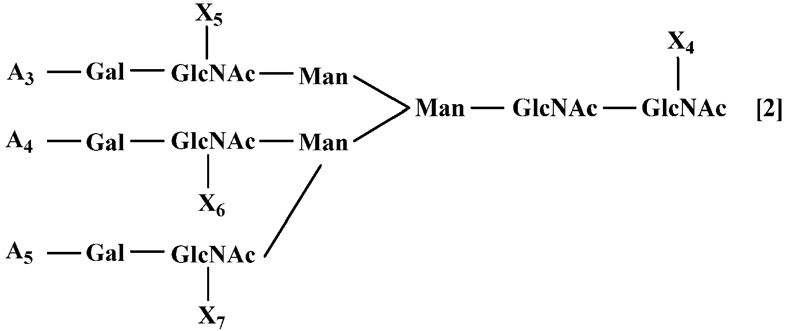
      (in the formula 2, X₄, X₅, X₆, and X₇ each independently represent Fuc or H, and A₃, A₄, and A₅ each independently represent Neu5Ac, Neu5Ac-Neu5Ac, or H).
[9] The reagent kit according to [8], in which the sugar chain is a sugar chain represented by any one of the following sugar chains (1-1) to (4-1),
   (1-1) at least one sugar chain represented by the formula 1 or 2 which is bound to an asparagine residue at position 70 from a N-terminal of an amino acid sequence of the alpha 1-antitrypsin;
   (2-1) a sugar chain represented by the formula 1 which is bound to an asparagine residue at position 79, an asparagine residue at position 186, or an asparagine residue at position 269 from a N-terminal of an amino acid sequence of the leucine-rich alpha 2-glycoprotein;
   (3-1) at least one sugar chain represented by the formula 1 or 2 which is bound to an asparagine residue at position 93 from a N-terminal of an amino acid sequence of the alpha 1-antichymotrypsin; and
   (4-1) a sugar chain represented by the formula 1 which is bound to an asparagine residue at position 415 from a N-terminal of an amino acid sequence of the complement component 9.

### Advantageous Effects of Invention

According to the biomarker, the method, and the reagent kit for assisting diagnosis of colon cancer of the present invention, it is possible to assist diagnosis of colon cancer with high accuracy.

### (Brief Description of Drawings)

Fig. 1 is a graph showing average values of colon cancer patients (n = 18) and average values of healthy subjects (n = 20) with respect to the peak surface area values of glycopeptides A, B, C, and D.
Fig. 2 is a graph showing average values of colon cancer patients (n = 18) and average values of healthy subjects (n = 20) with respect to the peak surface area values of glycopeptides E, F, G, and H.
Fig. 3 is a graph showing average values of colon cancer patients (n = 18) and average values of healthy subjects (n = 20) with respect to the peak surface area values of glycopeptides I, J, K, and L.
Fig. 4 is a graph showing an average value of colon cancer patients (n = 18) and an average value of healthy subjects (n = 20) with respect to the peak surface area value of glycopeptide M.

### Description of Embodiments

### Biomarker for assisting diagnosis of colon cancer of present invention

A biomarker for assisting diagnosis of colon cancer of the present invention (hereinafter, also referred to as the biomarker of the present invention) contains a sugar chain selected from the following sugar chains (1) to (4):
(1) at least one sugar chain represented by the following formula 1 or 2 which is present in alpha 1-antitrypsin (hereinafter, also referred to as the biomarker (1) of the present invention),
(2) a sugar chain represented by the following formula 1 which is present in leucine-rich alpha 2-glycoprotein (hereinafter, also referred to as the biomarker (2) of the present invention),
(3) at least one sugar chain represented by the following formula 1 or 2 which is present in alpha 1-antichymotrypsin (hereinafter, also referred to as the biomarker (3) of the present invention), and
(4) a sugar chain represented by the following formula 1 which is present in a complement component 9 (hereinafter, also referred to as the biomarker (4) of the present invention).

It should be noted that the biomarker of the present invention may contain any one or a plurality of biomarkers among the biomarkers (1) to (4) of the present invention.

Hereinafter, the biomarkers (1) to (4) of the present invention will be described below.

### Biomarker (1) of present invention

The biomarker (1) of the present invention contains at least one sugar chain represented by the following formula 1 or 2 which is present in alpha 1-antitrypsin. 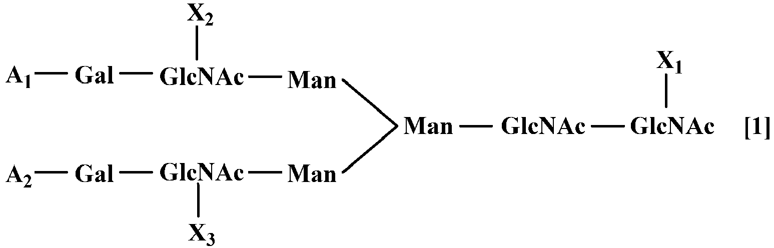
(In the formula 1, X₁, X₂, and X₃ each independently represent Fuc or H, and A₁ and A₂ each independently represent Neu5Ac, Neu5Ac-Neu5Ac, or H.) 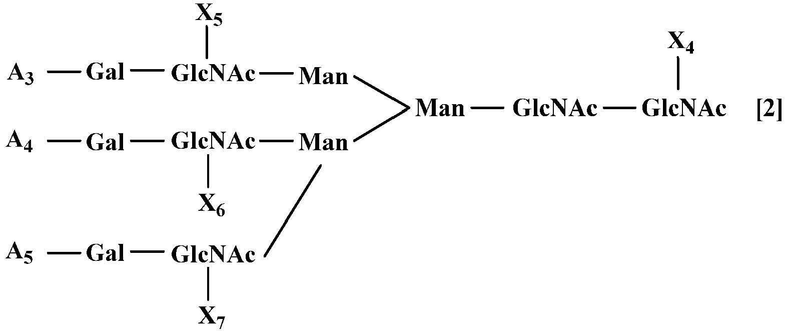
(In the formula 2, X₄, X₅, X₆, and X₇ each independently represent Fuc or H, and A₃, A₄, and A₅ each independently represent Neu5Ac, Neu5Ac-Neu5Ac, or H.)

In the formulae 1 and 2, Neu5Ac represents N-acetylneuraminic acid, Gal represents galactose, GlcNAc represents N-acetylglucosamine, Man represents mannose, Fuc represents fucose, and H represents a hydrogen atom.

In a case where X₁ in the formulae 1 and 2 represents H (the same applies to a case where X₂, X₃, X₄, X₅, X₆, and/or X₇ represents H), Xi-GlcNAc represents GlcNAc. In addition, in a case where the terminal A₁ in the formulae 1 and 2 represents H (the same applies to a case where A₂, A₃, A₄, and/or A₅ represents H), A₁-Gal- represents Gal-. Hereinafter, the same meanings are used in the present specification.

It should be noted that the biomarker (1) of the present invention may be a biomarker containing, among the sugar chains represented by the formula 1 and the sugar chains represented by the formula 2, only the sugar chains represented by the formula 1, only the sugar chains represented by the formula 2, or both the sugar chains represented by the formula 1 and the sugar chain represented by the formula 2. In addition, the biomarker may contain only a single sugar chain contained by the formula 1 or a plurality of sugar chains contained by the formula 1 or a single sugar chain contained by the formula 2 or a plurality of sugar chains represented by the formula 2.

The alpha 1-antitrypsin according to the biomarker (1) of the present invention is a glycoprotein consisting of 394 pieces of amino acids, which is an acute phase reactant that is mainly generated in hepatocytes and increases in blood during various inflammations. In addition, the alpha 1-antitrypsin is also known to function as the most major protease inhibitor in blood.

In addition, the amino acid sequence of the alpha 1-antitrypsin is registered in a database such as Uniprot (accession number: P01009).

The alpha 1- antitrypsin according to the biomarker (1) of the present invention contains variants that can be generated in a living body in addition to those registered in the database. For example, the variants and the like are generated by deletion, substitution, and/or addition of 1 to 5 pieces, preferably 1 or 2 pieces of amino acids in the alpha 1-antitrypsin, which is caused by polymorphism, mutation, or the like.

However, even in a case of a variant, a variant in which the amino acid residue (asparagine residue) at position 70 from the N-terminal of the amino acid sequence of the alpha 1-antitrypsin which is a binding site between at least one sugar chain represented by the formula 1 or 2 in the biomarker (1) of the present invention and the alpha 1-antitrypsin is conserved is preferable.

It is preferable that at least one sugar chain (terminal N-acetylglucosamine) represented by the formula 1 or 2 in the biomarker (1) of the present invention is bound (N-glycoside bond) to the amino acid residue (asparagine residue) at position 70 from the N-terminal of the amino acid sequence of alpha 1-antitrypsin.

Hereinafter, the sugar chain represented by the formula 1 or 2 in the biomarker (1) of the present invention will be described below.

### Sugar chain represented by formula 1 which is present in alpha 1-antitrypsin

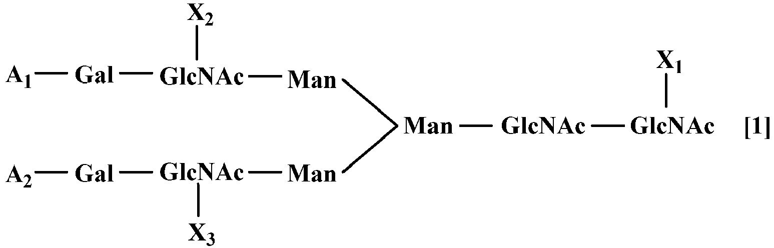

(In the formula 1, X₁, X₂, and X₃ each independently represent Fuc or H, and A₁ and A₂ each independently represent Neu5Ac, Neu5Ac-Neu5Ac, or H.)

As X₁, X₂, and X₃ in the formula 1, the combinations listed in Table 1 are preferable, and the combinations 4 to 7 are more preferable.

Table 1

As A₁ and A₂ in the formula 1, the combinations listed in Table 2 are preferable, and the combinations 1, 2, and 4 are more preferable.

Table 2

As the combinations of X₁, X₂, and X₃, and A₁ and A₂ in the formula 1, the combinations listed in Table 3 are preferable, and the combinations 16, 17, 19, 21, 22, 24, 26, 27, 29, 31, 32, and 34 are more preferable.

Table 3

As the sugar chain represented by the formula 1, for example, a sugar chain represented by the following formula 1' which is formed in a binding mode between monosaccharides such as GlcNAc, Man, Gal, Neu5Ac, and Fuc is preferable. 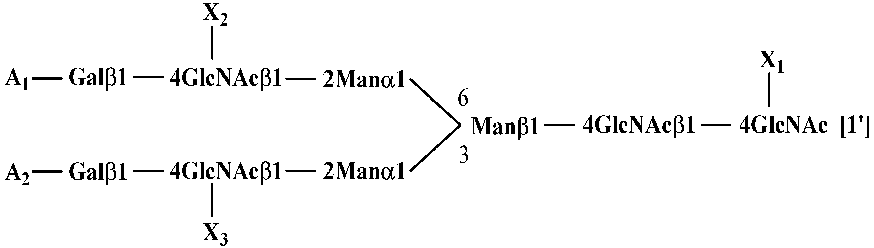 (In the formula 1', X₁, X₂, X₃, A₁, and A₂ each have the same definition as described above.)

Further, it is preferable that the formula 1" is represented by the following formula 1" or formula 1‴.

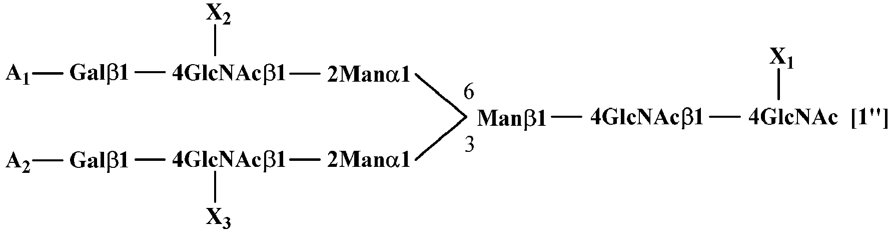

(In the formula 1", X₁, X₂, X₃, A₁, and A₂ each have the same definition as described above (here, any one of X₁, X₂, or X₃ represents Fuc, and the remaining two each represent H, the total number of Neu5Ac in A₁ and A₂ is 2.))

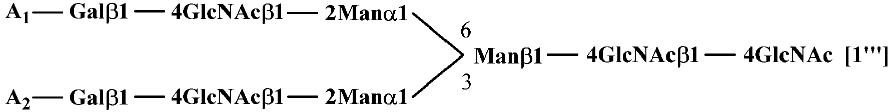

(In the formula 1‴, A₁ and A₂ each have the same definition as described above (here, the total number of Neu5Ac in A₁ and A₂ is 2.))

In a case where X₁, X₂, or X₃ in the formulae 1', 1", and 1‴ represents Fuc, it is preferable that Fuc binds to GlcNAc via an α1-6 glycoside bond. In a case where A₁ and/or A₂ represents Neu5Ac, it is preferable that Neu5Ac binds to Gal, for example, via an α2-3 glycoside bond or an α2-6 glycoside bond. In addition, in a case where A₁ or A₂ represents Neu5Ac-Neu5Ac, it is preferable that Neu5Ac binds to Neu5Ac, for example, via an α2-8 glycoside bond.

The specific examples and combinations of X₁, X₂, and X₃, and A₁ or A₂ in the formulae 1', 1", and 1‴ are the same as those of the formula 1.

### Sugar chain represented by formula 2 which is present in alpha 1-antitrypsin

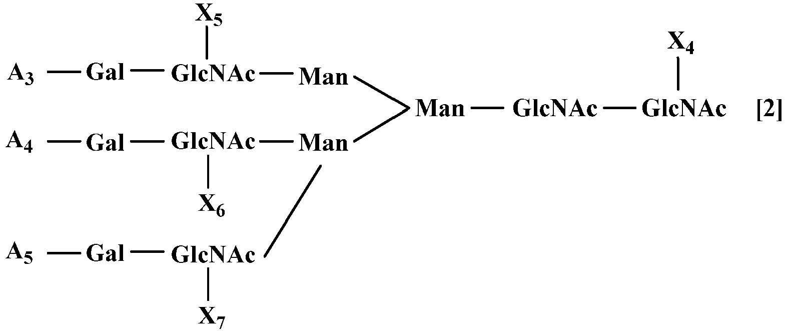

(In the formula 2, X₄, X₅, X₆, and X₇ each independently represent Fuc or H, A₃, A₄, and A₅ each independently represent Neu5Ac, Neu5Ac-Neu5Ac, or H.)

As X₄, X₅, X₆, and X₇ in the formula 2, for example, the combinations listed in Table 4 below are preferable.

Table 4

As A₃, A₄, and A₅ in the formula 2, the combinations listed in Table 5 are preferable.

Table 5

As the combinations of X₄, X₅, X₆, and X₇, and A₃, A₄, and A₅ in the formula 2, the combinations listed in Table 6 below are preferable.

Table 6

As the sugar chain represented by the formula 2, for example, a sugar chain represented by the following formula 2' which is formed in a binding mode between monosaccharides such as GlcNAc, Man, Gal, Neu5Ac, and Fuc is preferable. 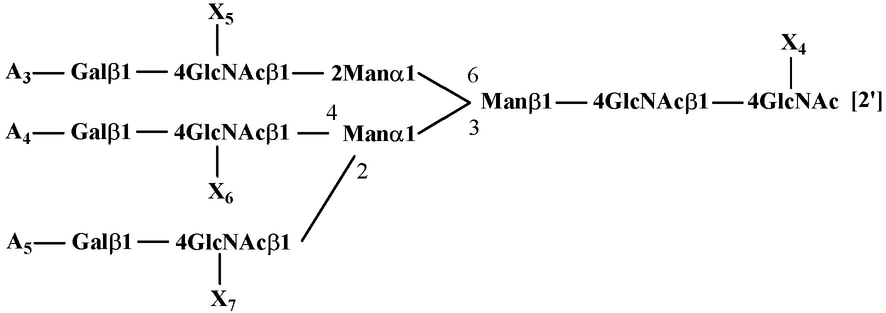 (In the formula 2', X₄, X₅, X₆, and X₇, A₃, A₄, and A₅ each have the same definition as described above.)

Further, it is preferable that the formula 2" is represented by the following formula 2" or formula 2"'. 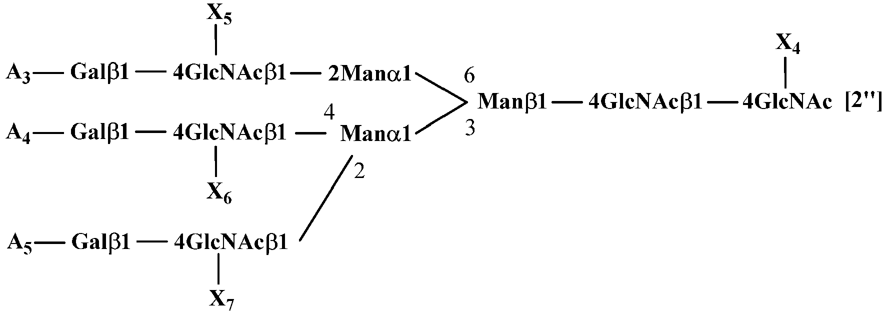 (In the formula 2", X₄, X₅, X₆, X₇, A₃, A₄, and A₅ each have the same definition as described above (here, any one of X₄, X₅, X₆, or X₇ represents Fuc, and the remaining three each represent H, and the total number of Neu5Ac in A₃, A₄, and A₅ is 2.))

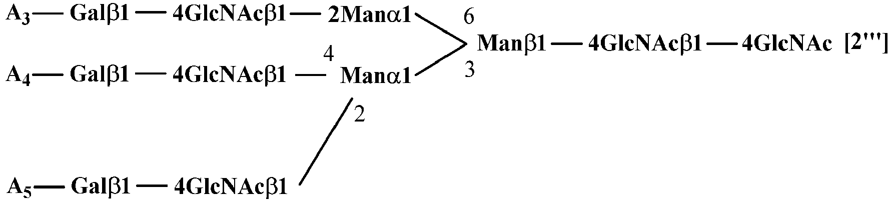

(In the formula 2"', A₃, A₄, and A₅ each have the same definition as described above (here, the total number of Neu5Ac in A₃, A₄, and A₅ is 2.))
In a case where X₄, X₅, X₆, or X₇ in the formulae 2', 2", and 2‴ represents Fuc, it is preferable that Fuc binds to GlcNAc via an α1-6 glycoside bond. In a case where A₃, A₄, and/or A₅ represents Neu5Ac, it is preferable that Neu5Ac binds to Gal, for example, via an α2-3 glycoside bond or an α2-6 glycoside bond. In addition, in a case where A₃, A₄, or A₅ represents Neu5Ac-Neu5Ac, it is preferable that Neu5Ac binds to Neu5Ac, for example, via an α2-8 glycoside bond.

The specific examples and combinations of X₄, X₅, X₆, X₇, A₃, A₄, and A₅ in the formulae 2', 2", and 2‴ are the same as those of the formula 2.

Examples of the biomarker (1) of the present invention, that is, the biomarker containing at least one sugar chain represented by the formula 1 or 2 which is present in alpha 1-antitrypsin include (i) at least one sugar chain represented by the formula 1 or 2 which is present in alpha 1-antitrypsin, (ii) alpha 1-antitrypsin to which at least one sugar chain represented by the formula 1 or 2 is bound, and (iii) peptide fragment which is a part of alpha 1-antitrypsin and to which at least one sugar chain represented by the formula 1 or 2 is bound. Among these, (ii) or (iii) is preferable.

It should be noted that the peptide fragment is an optional fragment containing at least the amino acid residue (asparagine residue) at position 70 from the N-terminal of the amino acid sequence of the alpha 1-antitrypsin, which is a binding site between at least one sugar chain represented by the formula 1 or 2 and the alpha 1-antitrypsin. Specific examples thereof include those produced in a living body and those produced as a result of subjecting alpha 1-antitrypsin to a protease treatment such as trypsin, lysyl endopeptidase, or AspN, and more specific examples thereof include those formed of 2 to 50 pieces of amino acid residues. Among these, a peptide fragment represented by SEQ ID NO: 1 is preferable.

### Biomarker of present invention (2)

The biomarker (2) of the present invention contains at least one sugar chain represented by the following formula 1 which is present in leucine-rich alpha 2-glycoprotein. 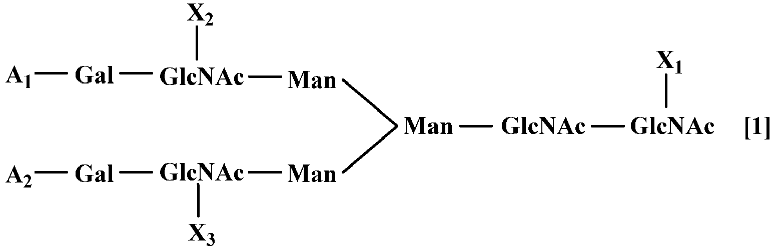 (in the formula 1, X₁, X₂, and X₃ each independently represent Fuc or H, and A₁ and A₂ each independently represent Neu5Ac, Neu5Ac-Neu5Ac, or H)

It should be noted that the biomarker (2) of the present invention may contain only a single sugar chain contained by the formula 1 or a plurality of sugar chains contained by the formula 1.

The leucine-rich alpha 2-glycoprotein according to the biomarker (2) of the present invention is a protein contained in blood and has a leucine-rich repeat structure. It has been reported that the leucine-rich alpha 2-glycoprotein is involved in various diseases such as rheumatoid arthritis and inflammatory bowel disease.

In addition, the amino acid sequence of the leucine-rich alpha 2-glycoprotein is registered in a database such as Uniprot (accession number: P02750).

The leucine-rich alpha 2-glycoprotein according to the biomarker (2) of the present invention contains variants that can be generated in a living body in addition to those registered in the database. For example, the variants and the like are generated by deletion, substitution, and/or addition of 1 to 5 pieces, preferably 1 or 2 pieces of amino acids in the leucine-rich alpha 2-glycoprotein, which is caused by polymorphism, mutation, or the like.

However, even in a case of a variant, a variant in which the amino acid residue (asparagine residue) at position 79, the amino acid residue (asparagine residue) at position 186, or the amino acid residue (asparagine residue) at position 269 from the N-terminal of the amino acid sequence of the leucine-rich alpha 2-glycoprotein which is a binding site between the sugar chain represented by the formula 1 in the biomarker (2) of the present invention and the leucine-rich alpha 2-glycoprotein is conserved is preferable.

It is preferable that the sugar chain (terminal N-acetylglucosamine) represented by the formula 1 in the biomarker (2) of the present invention is bound (N-glycoside bond) to the amino acid residue (asparagine residue) at position 79, the amino acid residue (asparagine residue) at position 186, or the amino acid residue (asparagine residue) at position 269 from the N-terminal of the amino acid sequence of the leucine-rich alpha 2-glycoprotein.

Hereinafter, the sugar chain represented by the formula 1 in the biomarker (2) of the present invention will be described below.

### Sugar chain represented by formula 1 which is present in leucine-rich alpha 2-glycoprotein

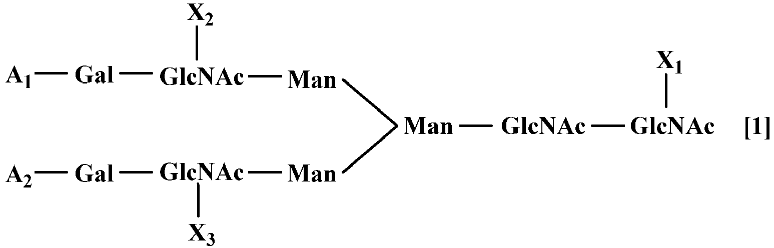

(in the formula 1, X₁, X₂, and X₃ each independently represent Fuc or H, and A₁ and A₂ each independently represent Neu5Ac, Neu5Ac-Neu5Ac, or H)

As X₁, X₂, and X₃ in the formula 1, the combinations listed in Table 1 are preferable.

As A₁ and A₂ in the formula 1, the combinations listed in Table 2 are preferable.

As the combinations of X₁, X₂, and X₃, and A₁ and A₂ in the formula 1, the combinations listed in Table 3 are preferable.

As the sugar chain represented by the formula 1, for example, a sugar chain represented by the following formula 1' which is formed in a binding mode between monosaccharides such as GlcNAc, Man, Gal, Neu5Ac, and Fuc is preferable.

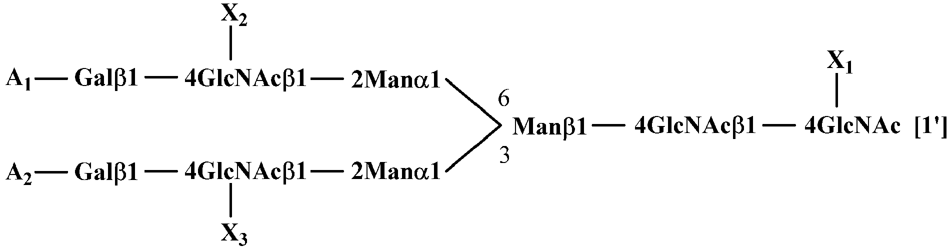

(In the formula 1', X₁, X₂, X₃, A₁, and A₂ each have the same definition as described above.)

Further, it is preferable that the formula 1' is represented by the following formula 1", formula 1‴, or formula 1ʺʺ.

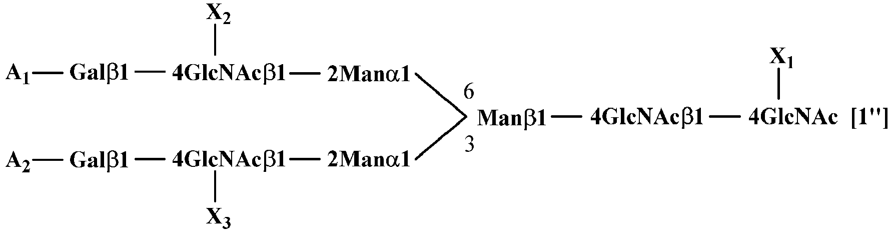

(In the formula 1", X₁, X₂, X₃, A₁, and A₂ each have the same definition as described above (here, any one of X₁, X₂, or X₃ represents Fuc, and the remaining two each represent H, the total number of Neu5Ac in A₁ and A₂ is 2.))

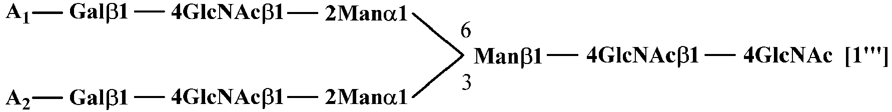

(In the formula 1‴, A₁ and A₂ each have the same definition as described above (here, the total number of Neu5Ac in A₁ and A₂ is 2.))

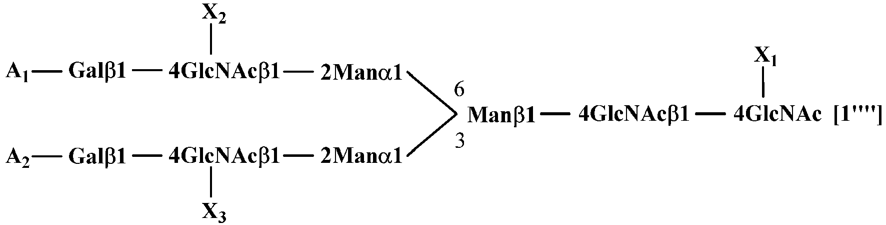

(In the formula 1", X₁, X₂, X₃, A₁, and A₂ each have the same definition as described above (here, any two of X₁, X₂, and X₃ each represent Fuc, and the remaining one represents H, and the total number of Neu5Ac in A₁ and A₂ is 1.))

In a case where X₁, X₂, and X₃ in the formulae 1', 1", 1‴, and 1"" represent Fuc, it is preferable that Fuc binds to GlcNAc via an α1-6 glycoside bond. In a case where A₁ and/or A₂ represents Neu5Ac, it is preferable that Neu5Ac binds to Gal, for example, via an α2-3 glycoside bond or an α2-6 glycoside bond. In addition, in a case where A₁ or A₂ represents Neu5Ac-Neu5Ac, it is preferable that Neu5Ac binds to Neu5Ac, for example, via an α2-8 glycoside bond.

The specific examples and combinations of X₁, X₂, and X₃, and A₁ or A₂ in the formulae 1', 1", and 1‴ are the same as those of the formula 1.

Examples of the biomarker (2) of the present invention, that is, the biomarker containing the sugar chain represented by the formula 1 which is present in leucine-rich alpha 2-glycoprotein include (i) a sugar chain represented by the formula 1 which is present in leucine-rich alpha 2-glycoprotein, (ii) leucine-rich alpha 2-glycoprotein to which a sugar chain represented by the formula 1 is bound, and (iii) a peptide fragment which is a part of leucine-rich alpha 2-glycoprotein and to which a sugar chain represented by the formula 1 is bound. Among these, (ii) or (iii) is preferable.

It should be noted that the peptide fragment is an optional fragment containing at least the amino acid residue (asparagine residue) at position 70, the amino acid residue (asparagine residue) at position 186, or the amino acid residue (asparagine residue) at position 269 from the N-terminal of the amino acid sequence of the leucine-rich alpha 2-glycoprotein, which is a binding site between the sugar chain represented by the formula 1 and the leucine-rich alpha 2-glycoprotein. Specific examples thereof include those produced in a living body and those produced as a result of subjecting alpha 1-antitrypsin to a protease treatment such as trypsin, lysyl endopeptidase, or AspN, and more specific examples thereof include those formed of 2 to 50 pieces of amino acid residues. Among these, a peptide fragment represented by SEQ ID NO: 2, 3, or 4 is preferable.

### Biomarker of present invention (3)

The biomarker (3) of the present invention contains at least one sugar chain represented by the following formula 1 or 2 which is present in alpha 1-antichymotrypsin. 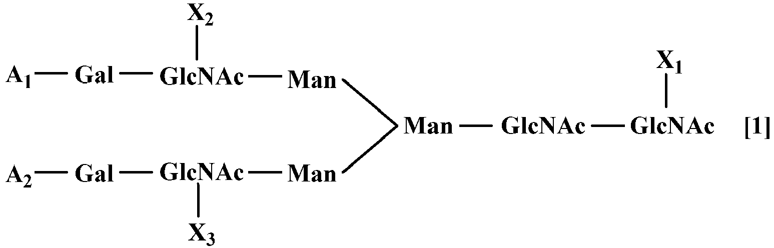
(in the formula 1, X₁, X₂, and X₃ each independently represent Fuc or H, and A₁ and A₂ each independently represent Neu5Ac, Neu5Ac-Neu5Ac, or H) 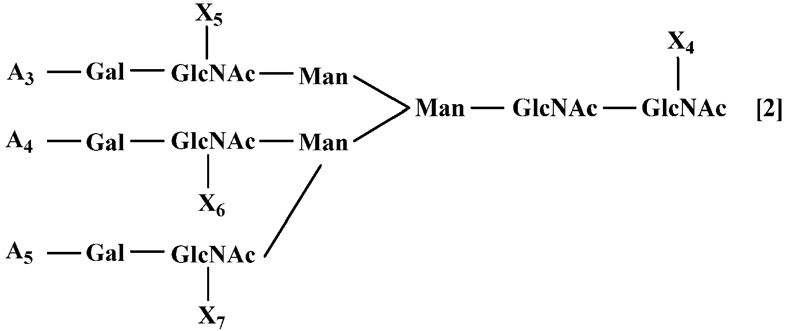
(in the formula 2, X₄, X₅, X₆, and X₇ each independently represent Fuc or H, and A₃, A₄, and A₅ each independently represent Neu5Ac, Neu5Ac-Neu5Ac, or H).

It should be noted that the biomarker (3) of the present invention may be a biomarker containing, among the sugar chains represented by the formula 1 and the sugar chains represented by the formula 2, only the sugar chains represented by the formula 1, only the sugar chains represented by the formula 2, or both the sugar chains represented by the formula 1 and the sugar chain represented by the formula 2. In addition, the biomarker may contain only a single sugar chain contained by the formula 1 or a plurality of sugar chains contained by the formula 1 or a single sugar chain contained by the formula 2 or a plurality of sugar chains represented by the formula 2.

The alpha 1-antichymotrypsin according to the biomarker (3) of the present invention is a glycoprotein consisting of 433 pieces amino acids and is one of the serine protease inhibitor families present in serum. In addition, alpha 1-antichymotrypsin is also known to function as the most major protease inhibitor in blood.

In addition, the amino acid sequence of the alpha 1-antichymotrypsin is registered in a database such as Uniprot (accession number: P01011).

The alpha 1-antichymotrypsin according to the biomarker (3) of the present invention contains variants that can be generated in a living body in addition to those registered in the database. For example, the variants the like are generated by deletion, substitution, and/or addition of 1 to 5 pieces, preferably 1 or 2 pieces of amino acids in the alpha 1-antichymotrypsin, which is caused by polymorphism, mutation, or the like.

However, even in a case of a variant, a variant in which the amino acid residue (asparagine residue) at position 93 from the N-terminal of the amino acid sequence of the alpha 1-antichymotrypsin which is a binding site between at least one sugar chain represented by the formula 1 or 2 in the biomarker (3) of the present invention and the alpha 1-antichymotrypsin is conserved is preferable.

It is preferable that at least one sugar chain (terminal N-acetylglucosamine) represented by the formula 1 or 2 in the biomarker (3) of the present invention is bound (N-glycoside bond) to the amino acid residue (asparagine residue) at position 93 from the N-terminal of the amino acid sequence of the alpha 1-antichymotrypsin.

Hereinafter, the sugar chain represented by the formula 1 or 2 in the biomarker (3) of the present invention will be described below.

### Sugar chain represented by formula 1 which is present in alpha 1-antichymotrypsin

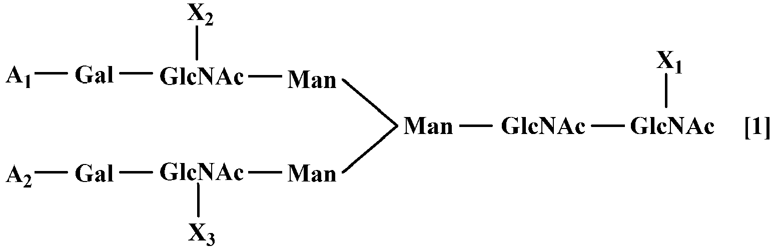

(in the formula 1, X₁, X₂, and X₃ each independently represent Fuc or H, and A₁ and A₂ each independently represent Neu5Ac, Neu5Ac-Neu5Ac, or H)

As the combinations of X₁, X₂, and X₃ in the formula 1, the combinations listed in Table 1 are preferable, and the combinations 4 to 7 are more preferable.

As the combinations of A₁ and A₂ in the formula 1, the combinations listed in Table 2 are preferable, and the combinations 1, 2, and 4 are more preferable.

As the combinations of X₁, X₂, and X₃, and A₁ and A₂ in the formula 1, the combinations listed in Table 3 are preferable, and the combinations 16, 17, 19, 21, 22, 24, 26, 27, 29, 31, 32, and 34 are more preferable.

As the sugar chain represented by the formula 1, for example, a sugar chain represented by the following formula 1' which is formed in a binding mode between monosaccharides such as GlcNAc, Man, Gal, Neu5Ac, and Fuc is preferable. 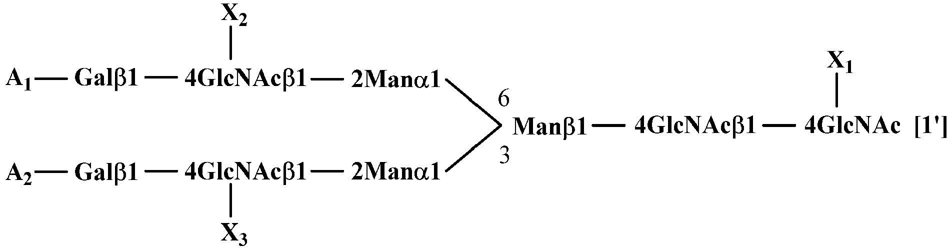 (In the formula 1', X₁, X₂, X₃, A₁, and A₂ each have the same definition as described above.)

Further, it is preferable that the formula 1" is represented by the following formula 1" or formula 1‴. 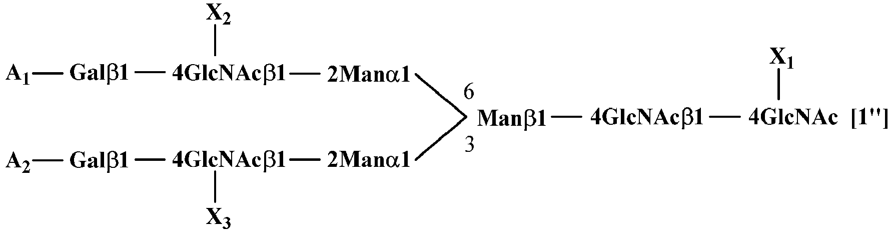 (In the formula 1", X₁, X₂, X₃, A₁, and A₂ each have the same definition as described above (here, any one of X₁, X₂, or X₃ represents Fuc, and the remaining two each represent H, the total number of Neu5Ac in A₁ and A₂ is 2.)) 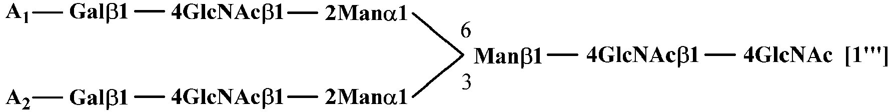 (In the formula 1‴, A₁ and A₂ each have the same definition as described above (here, the total number of Neu5Ac in A₁ and A₂ is 2.))

In a case where X₁, X₂, or X₃ in the formulae 1', 1", and 1‴ represents Fuc, it is preferable that Fuc binds to GlcNAc via an α1-6 glycoside bond. In a case where A₁ and/or A₂ represents Neu5Ac, it is preferable that Neu5Ac binds to Gal, for example, via an α2-3 glycoside bond or an α2-6 glycoside bond. In addition, in a case where A₁ or A₂ represents Neu5Ac-Neu5Ac, it is preferable that Neu5Ac binds to Neu5Ac, for example, via an α2-8 glycoside bond.

The specific examples and combinations of X₁, X₂, and X₃, and A₁ or A₂ in the formulae 1', 1", and 1‴ are the same as those of the formula 1.

### Sugar chain represented by formula 2 which is present in alpha 1-antichymotrypsin

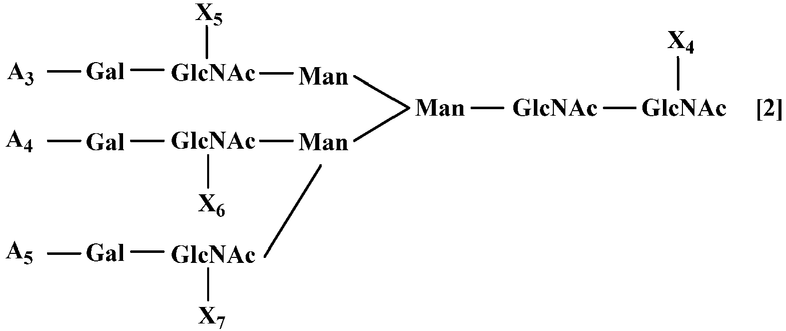

(in the formula 2, X₄, X₅, X₆, and X₇ each independently represent Fuc or H, and A₃, A₄, and A₅ each independently represent Neu5Ac, Neu5Ac-Neu5Ac, or H).

As X₄, X₅, X₆, and X₇ in the formula 2, the combinations listed in Table 4 are preferable.

As A₃, A₄, and A₅ in the formula 2, the combinations listed in Table 5 are preferable.

As the combinations of X₄, X₅, X₆, and X₇, and A₃, A₄, and A₅ in the formula 2, the combinations listed in Table 6 are preferable.

As the sugar chain represented by the formula 2, for example, a sugar chain represented by the following formula 2' which is formed in a binding mode between monosaccharides such as GlcNAc, Man, Gal, Neu5Ac, and Fuc is preferable. 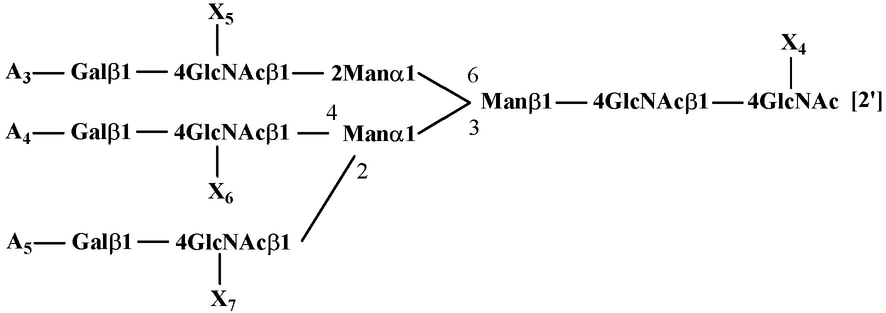 (In the formula 2', X₄, X₅, X₆, and X₇, A₃, A₄, and A₅ each have the same definition as described above.)

Further, it is preferable that the formula 2" is represented by the following formula 2" or formula 2"'. 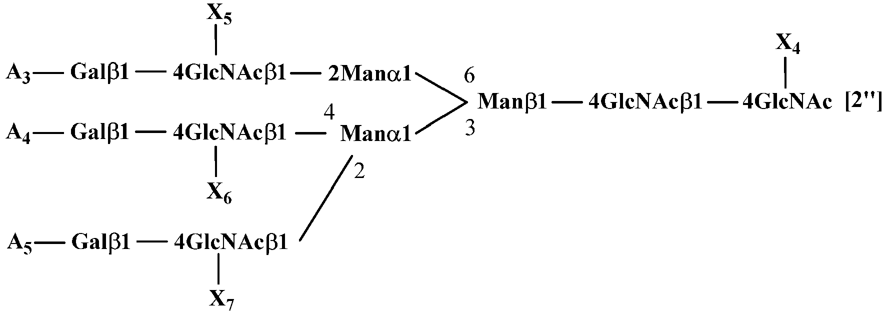 (In the formula 2", X₄, X₅, X₆, and X₇, A₃, A₄, and A₅ each have the same definition as described above (here, any one of X₄, X₅, X₆, or X₇ represents Fuc, and the remaining three each represent H, and the total number of Neu5Ac in A₃, A₄, and A₅ is 2.)) 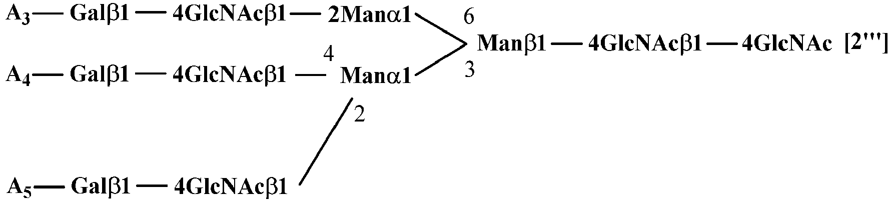 (In the formula 2"', A₃, A₄, and A₅ each have the same definition as described above (here, the total number of Neu5Ac in A₃, A₄, and A₅ is 2.))

In a case where X₄, X₅, X₆, or X₇ in the formulae 2', 2", and 2‴ represents Fuc, it is preferable that Fuc binds to GlcNAc via an α1-6 glycoside bond. In a case where A₃, A₄, and A₅ represent Neu5Ac, it is preferable that Neu5Ac binds to Gal, for example, via an α2-3 glycoside bond or an α2-6 glycoside bond. In addition, in a case where A₁ or A₂ represents Neu5Ac-Neu5Ac, it is preferable that Neu5Ac binds to Neu5Ac, for example, via an α2-8 glycoside bond.

The specific examples and combinations of X₄, X₅, X₆, and X₇, and A₃, A₄, and A₅ in the formulae 2', 2", and 2‴ are the same as those of the formula 1.

Examples of the biomarker (3) of the present invention, that is, the biomarker containing at least one sugar chain represented by the formula 1 or 2 which is present in alpha 1-antichymotrypsin include (i) at least one sugar chain represented by the formula 1 or 2 which is present in alpha 1-antichymotrypsin, (ii) alpha 1-antichymotrypsin to which at least one sugar chain represented by the formula 1 or 2 is bound, and (iii) peptide fragment which is a part of alpha 1-antichymotrypsin and to which at least one sugar chain represented by the formula 1 or 2 is bound. Among these, (ii) or (iii) is preferable.

It should be noted that the peptide fragment is an optional fragment containing at least the amino acid residue (asparagine residue) at position 93 from the N-terminal of the amino acid sequence of the alpha 1-antichymotrypsin, which is a binding site between at least one sugar chain represented by the formula 1 or 2 and the alpha 1-antichymotrypsin. Specific examples thereof include those produced in a living body and those produced as a result of subjecting alpha 1-antichymotrypsin to a protease treatment such as trypsin, lysyl endopeptidase, or AspN, and more specific examples thereof include those formed of 2 to 50 pieces of amino acid residues. Among these, a peptide fragment represented by SEQ ID NO: 5 is preferable.

### Biomarker of present invention (4)

The biomarker (4) of the present invention contains a sugar chain represented by the following formula 1 which is present in the complement component 9. 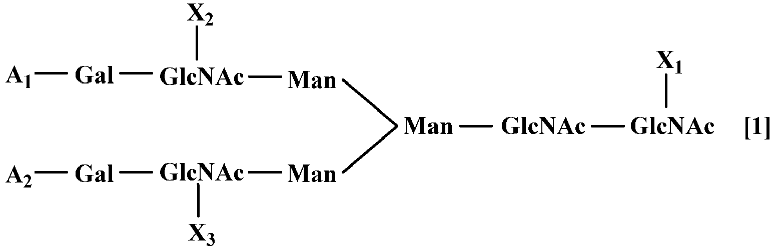 (in the formula 1, X₁, X₂, and X₃ each independently represent Fuc or H, and A₁ and A₂ each independently represent Neu5Ac, Neu5Ac-Neu5Ac, or H)

The complement component 9 according to the biomarker (4) of the present invention is one of the proteins that assist an immune reaction (antigen-antibody reaction). Known physiological functions include opsonization of antigens, destruction of bacteria by membrane attack complexes, and activation of phagocytes such as macrophages.

In addition, the amino acid sequence of the complement component 9 is registered in a database such as Uniprot (accession number: P02748).

The complement component 9 according to the biomarker (4) of the present invention contains variants and the like that can be generated in a living body in addition to those registered in the database. For example, the variants the like are generated by deletion, substitution, and/or addition of 1 to 5 pieces, preferably 1 or 2 pieces of amino acids in the complement component 9, which is caused by polymorphism, mutation, or the like.

However, even in a case of a variant, a variant in which the amino acid residue (asparagine residue) at position 415 from the N-terminal of the amino acid sequence of the complement component 9 which is a binding site between the sugar chain represented by the formula 1 in the biomarker (4) of the present invention and the complement component 9 is conserved is preferable.

It is preferable that the sugar chain (terminal N-acetylglucosamine) represented by the formula 1 in the biomarker (4) of the present invention is bound (N-glycoside bond) to the amino acid residue (asparagine residue) at position 415 from the N-terminal of the amino acid sequence of the complement component 9.

Hereinafter, the sugar chains represented by the formula 1 in the biomarker (4) of the present invention will be described.

### Sugar chain represented by formula 1 which is present in complement component 9

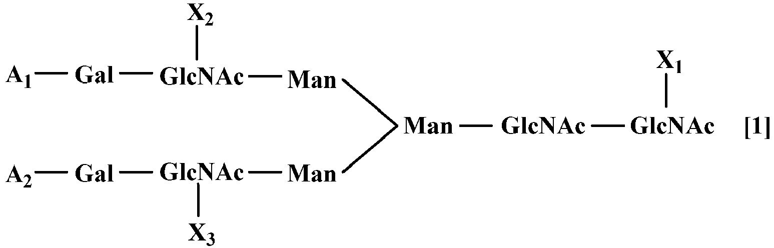

(In the formula 1, X₁, X₂, and X₃ each independently represent Fuc or H, and A₁ and A₂ each independently represent Neu5Ac, Neu5Ac-Neu5Ac, or H.)

As the combinations of X₁, X₂, and X₃ in the formula 1, the combinations listed in Table 1 are preferable, and the combination 7 is more preferable.

As the combinations of A₁ and A₂ in the formula 1, the combinations listed in Table 2 are preferable, and the combinations 1, 2, and 4 are more preferable.

As the combinations of X₁, X₂, and X₃, and A₁ and A₂ in the formula 1, the combinations listed in Table 3 are preferable, and the combinations 31, 32, and 34 are more preferable.

As the sugar chain represented by the formula 1, for example, a sugar chain represented by the following formula 1' which is formed in a binding mode between monosaccharides such as GlcNAc, Man, Gal, Neu5Ac, and Fuc is preferable. 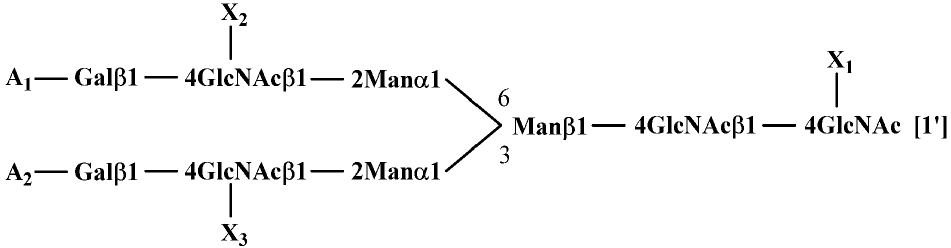 (In the formula 1', X₁, X₂, X₃, A₁, and A₂ each have the same definition as described above.)

Further, it is preferable that the formula 1' is represented by the following formula 1‴. 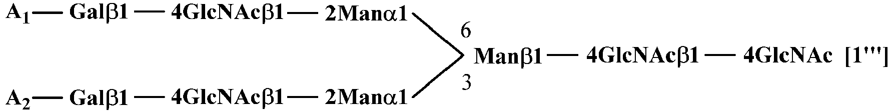 (In the formula 1‴, A₁ and A₂ each have the same definition as described above (here, the total number of Neu5Ac in A₁ and A₂ is 2.))

In a case where X₁, X₂, or X₃ in the formula 1' represents Fuc, it is preferable that Fuc binds to GlcNAc via an α1-6 glycoside bond. In a case where A₁ and/or A₂ in the formulae 1' and 1‴ represents Neu5Ac, it is preferable that Neu5Ac binds to Gal, for example, via an α2-3 glycoside bond or an α2-6 glycoside bond. In addition, in a case where A₁ or A₂ represents Neu5Ac-Neu5Ac, it is preferable that Neu5Ac binds to Neu5Ac, for example, via an α2-8 glycoside bond.

The specific examples and combinations of X₁, X₂, or X₃, and A₁ or A₂ in the formulae 1' and 1‴ are the same as those of the formula 1.

Examples of the biomarker (4) of the present invention, that is, the biomarker containing a sugar chain represented by the formula 1 which is present in the complement component 9 include (i) a sugar chain represented by the formula 1 which is present in the complement component 9, (ii) a complement component 9 to which a sugar chain represented by the formula 1 is bound, and (iii)a peptide fragment which is a part of the complement component 9 and to which a sugar chain represented by the formula 1 is bound. Among these, (ii) or (iii) is preferable.

It should be noted that the peptide fragment is an optional fragment containing at least the amino acid residue (asparagine residue) at position 415 from the N-terminal of the amino acid sequence of the complement component 9, which is a binding site between the sugar chain represented by the formula 1 and the complement component 9. Specific examples thereof include those produced in a living body and those produced as a result of subjecting the complement component 9 to a protease treatment such as trypsin, lysyl endopeptidase, or AspN, and more specific examples thereof include those formed of 2 to 50 pieces of amino acid residues. Among these, a peptide fragment represented by SEQ ID NO: 6 is preferable.

As the biomarker of the present invention, (i) biomarker containing at least one sugar chain represented by the formula 1 or 2 which is present in alpha 1-antitrypsin or (ii) biomarker containing a sugar chain represented by the formula 1 which is present in leucine-rich alpha 2-glycoprotein is preferable, (i) biomarker containing at least one sugar chain represented by the formula 1 or 2 which is present in alpha 1-antitrypsin is more preferable, and (iii) at least one sugar chain represented by the formula 1 or 2 which is bound to the amino acid residue (asparagine residue) at position 70 from the N-terminal of the amino acid sequence of alpha 1-antitrypsin is particularly preferable.

### Colon cancer according to present invention

The colon cancer according to the present invention is a cancer (malignant tumor) originating from the large intestine. Examples of the colon cancer according to the present invention includes colon carcinoma and rectal cancer.

### Method of assisting diagnosis of colon cancer of present invention

A method of assisting diagnosis of colon cancer of the present invention (hereinafter, also referred to as the assisting method of the present invention) includes measuring a sugar chain selected from the following sugar chains (1) to (4) in a sample derived from a test animal (hereinafter, also referred to as a measuring step according to the present invention) and determining whether the test animal has colon cancer based on obtained measurement results (hereinafter, also referred to as a determining step according to the present invention).
(1) At least one sugar chain represented by the formula 1 or 2 which is present in alpha 1-antitrypsin (the biomarker (1) of the present invention),
(2) a sugar chain represented by the formula 1 which is present in leucine-rich alpha 2-glycoprotein (the biomarker (2) of the present invention),
(3) at least one sugar chain represented by the formula 1 or 2 which is present in alpha 1-antichymotrypsin (the biomarker (3) of the present invention), and
(4) a sugar chain represented by the formula 1 which is present in the complement component 9 (the biomarker (4) of the present invention)

It should be noted that the assisting method of the present invention may be performed by using any one of the biomarkers (1) to (4) of the present invention or a plurality of kinds from among the biomarkers (1) to (4) of the present invention.

### Test animal according to present invention

Examples of the test animal according to the present invention include mammals such as humans, monkeys, mice, rats, dogs, cats, pigs, rabbits, and chimpanzees. Among these, humans, monkeys, mice, or rats are preferable, and humans are more preferable.

### Sample of present invention

The sample according to the present invention may be derived from the test animals, and examples thereof include biological samples such as serum, plasma, whole blood, urine, saliva, cerebrospinal fluid, interstitial fluid, sweat, tears, amnionic fluid, bone marrow fluid, pleural effusion, ascites, synovial fluid, aqueous humor, and vitreous humor. Among these, blood-derived samples such as serum, plasma, and whole blood are preferable, and serum is more preferable.

A method of obtaining (collecting) the sample from the test animal according to the present invention is not particularly limited, and for example, the sample may be obtained (collected) from the test animal by a method known per se, which means that the sample may be obtained (collected) by performing separation, concentration, purification, and the like using a method known per se.

It should be noted that the sample may be a sample immediately after being obtained (collected) from the test animal or may be a sample conserving the sample. A method of conserving the sample is not limited as long as the method is typically used in this field.

### Measuring step according to present invention

The measuring step according to the present invention is performed by measuring a sugar chain selected from the following sugar chains (1) to (4).
(1) At least one sugar chain represented by the formula 1 or 2 which is present in alpha 1-antitrypsin,
(2) a sugar chain represented by the formula 1 which is present in leucine-rich alpha 2-glycoprotein,
(3) at least one sugar chain represented by the formula 1 or 2 which is present in alpha 1-antichymotrypsin, and
(4) a sugar chain represented by the following formula 1 which is present in the complement component 9

It should be noted that the measuring step according to the present invention may be performed by measuring any one of the biomarkers (1) to (4) of the present invention or by measuring a plurality of kinds from among the biomarkers (1) to (4).

The measurement of the sugar chain selected from the sugar chains (1) to (4) in the measuring step according to the present invention is measurement of one biomarker selected from the biomarkers (1) to (4) of the present invention, and specific examples thereof include (i) measurement of the amount of the sugar chain itself bound to (derived from) the core protein in the biomarker of the present invention, (ii) measurement of the amount of the core protein to which the sugar chain in the biomarker of the present invention is bound, and (iii) measurement of the amount of the peptide fragment which is a part of the core protein in the biomarker of the present invention and to which a sugar chain is bound. Among these, the measurement (ii) or (iii) is preferable.

The core protein in the biomarker of the present invention denotes a part other than the sugar chain in the biomarker of the present invention, and the core protein is alpha 1-antitrypsin in a case of the biomarker (1) of the present invention, the core protein is leucine-rich alpha 2-glycoprotein in a case of the biomarker (2) of the present invention, the core protein is alpha 1-antichymotrypsin in a case of the biomarker (3) of the present invention, and the core protein is the complement component 9 in a case of the biomarker (4) of the present invention.

It should be noted that "amount" may be any of an absolute value such as the volume or the mass or a relative value such as a value calculated from the concentration, the ion intensity, the absorbance, the fluorescence intensity, the turbidity, or the peak surface area.

The measurement method in the measuring step according to the present invention is not limited as long as the method is typically used in this field, and specific examples thereof include (A) method of using a substance (such as an antibody or a lectin) having an affinity for the biomarker of the present invention and (B) method of using a mass spectrometry method. Among these, the method (A) is preferable.

Hereinafter, each of the methods (A) and (B) will be described in detail.

### (A) Method of using substance having affinity for biomarker of present invention

Examples of the substance having an affinity for the biomarker of the present invention include an antibody that specifically binds to a core protein (or a peptide fragment) in the biomarker of the present invention and a lectin or an antibody that specifically binds to a sugar chain in the biomarker of the present invention.

The biomarker of the present invention may be measured by an immunological measurement method in a case where only an antibody is used as the substance having an affinity for the biomarker of the present invention and the biomarker of the present invention may be measured by a method in conformity with an immunological measurement method in a case where a substance other than an antibody (for example, a lectin), or a substance other than an antibody and an antibody are used. Examples of the principle of these measurement methods include a sandwich method and a competition method, and a homogenous measurement system or a heterogeneous measurement system may be used.

As the method of using the substance having an affinity for the biomarker of the present invention, specifically, for example, the amount of the biomarker of the present invention may be measured by bringing the substance having an affinity for the biomarker of the present invention and the biomarker of the present invention into contact with each other to form a complex of the substance having an affinity for the biomarker of the present invention and the biomarker of the present invention.

Specific examples of the method using the substance having an affinity for the biomarker of the present invention include an immunoturbidmetry method such as an enzyme-linked immunosorbent assay method (ELISA method), an enzyme immunoassay method (EIA method), a radioimmunoassay method (RIA method), a fluorescence immunoassay method (FIA method), a chemiluminescence enzyme immunoassay method (CLEIA method), or a latex coagulating nephelometry method, an immononephelometry method, an immunochromatography method, a western blotting method, luminescent oxygen channeling immunoassay (LOCI method), liquid-phase binding assay-electrokinetic analyte transport assay (LBA-EATA method), a capillary electrophoresis method such as a lectin electrophoresis method, a surface plasmon resonance method, and a lectin column method. Among these, an ELISA method, an EIA method, an RIA method, an FIA method, a CLEIA method, an LBA-EATA method, or a capillary electrophoresis method such as a lectin electrophoresis method is more preferable.

Specific examples of the antibody that specifically binds to a core protein (or a peptide fragment) in the biomarker of the present invention include an anti-alpha 1-antitrypsin antibody in a case of the biomarker (1) of the present invention, an anti-leucine-rich alpha 2-glycoprotein antibody in a case of the biomarker (2) of the present invention, an anti-alpha 1-antichymotrypsin antibody in a case of the biomarker (3) of the present invention, and an anti-complement component 9 antibody in a case of the biomarker (4) of the present invention.

The antibody may be any of a polyclonal antibody or a monoclonal antibody, and these may be used alone or in combination.

The antibody may be an antibody fragment such as Fab, F(ab'2), Fv, or sFv, or a synthetic antibody such as a diabody, a triabody, or a tetrabody.

In addition, as the antibody, a commercially available antibody or an antibody prepared by a method known per se may be used.

It should be noted that an antibody may be prepared, for example, by a method described in "The Immunoassay" (edited by Biochemical Assay Society of Japan, Kodansha, 2014) in a case where the antibody is prepared by a method known per se.

In addition, the antibody may be labeled with a labeling substance. Specific examples of the labeling substance include enzymes such as horseradish peroxidase (HRP), bovine small intestinal alkaline phosphatase, and β-galactosidase, radioisotopes such as ^{99m}Tc, ¹³¹I, ¹²⁵I, ¹⁴C, ³H, ³²P, and ³⁵S, fluorescent substances such as fluorescein, fluorescein isothiocyanate (FITC), 4-methylumbelliferone, rhodamine, and derivatives thereof, luminescent substances such as luciferin, luminol, and ruthenium complexes, substances having absorption in an ultraviolet region such as phenol, naphthol, anthracene, and derivatives thereof, substances having properties as a spin labeling agent represented by a compound containing an oxyl group such as 4-amino-2,2,6,6-tetramethylpiperidin-1-oxyl, coloring agents such as HiLyte-based coloring agents, Alexa-based coloring agents, and CyDye-based coloring agents, and nanoparticles such as gold colloids and quantum dots.

It should be noted that a method known per se may be used as a method of binding the labeling substance to an antibody.

Specific examples of the lectin that specifically binds to the sugar chain in the biomarker of the present invention are as follows.

That is, the examples of the lectin include, in a case of the biomarker (1) of the present invention, Aleuria Aurantia Lectin (AAL) or Lotus Tetragonolobus Lectin (LTL) that binds to the Fuc (fucose) of the sugar chain represented by the formula 1, Elderberry Balk Lectin (EBL) or Maackia Amurensis Lectin (MAA) that binds to Neu5Ac (N-acetylneuraminic acid), AAL or LTL that binds to Fuc (fucose) of the sugar chain represented by the formula 2, and EBL or MAA that binds to Neu5Ac (N-acetylneuraminic acid).

Examples thereof include, in a case of the biomarker (2) of the present invention, AAL or LTL that binds to Fuc (fucose) of the sugar chain represented by the formula 1, and EBL or MAA that binds to Neu5Ac (N-acetylneuraminic acid).

Examples thereof include, in a case of the biomarker (3) of the present invention include AAL or LTL that binds to Fuc (fucose) of the sugar chain represented by the formula 1, and EBL or MAA that binds to Neu5Ac (N-acetylneuraminic acid).

In addition, examples thereof include, in a case of the biomarker (4) of the present invention, AAL or LTL that binds to Fuc (fucose) of the sugar chain represented by the formula 1, and EBL or MAA that binds to Neu5Ac (N-acetylneuraminic acid).

The lectin may be labeled with a labeling substance, and the labeling substance is as described in the section of the antibody that specifically binds to the core protein (or the peptide fragment) in the biomarker of the present invention, and the specific examples thereof are the same as described above. In addition, a method known per se may be used as a method of binding the labeling substance to the lectin.

Specific examples of the antibody that specifically binds to the sugar chain in the biomarker of the present invention are as follows.

That is, examples of the antibody include, in a case of the biomarker (1) of the present invention, an antibody that specifically binds to the sugar chain represented by the formula 1 and an antibody that specifically binds to the sugar chain represented by the formula 2.

Examples thereof include, in a case of the biomarker (2) of the present invention, an antibody that specifically binds to the sugar chain represented by the formula 1.

Examples thereof include, in a case of the biomarker (3) of the present invention, an antibody that specifically binds to the sugar chain represented by the formula 1 and an antibody that specifically binds to the sugar chain represented by the formula 2.

Examples thereof include, in a case of the biomarker (4) of the present invention, an antibody that specifically binds to the sugar chain represented by the formula 1.

The antibody may be labeled with a labeling substance, and the labeling substance is as described in the section of the antibody that specifically binds to the protein (or the peptide fragment) in the biomarker of the present invention, and specific examples thereof are the same as described above.

In addition, a method known per se may be used as a method of binding the labeling substance to the lectin.

The method of measuring the amount of the complex of the substance having an affinity for the biomarker of the present invention and the biomarker of the present invention may be any method as long as the amount of the complex can be measured by the method, and specific examples thereof include a method of detecting a signal derived from a labeling substance bound to a substance having an affinity for the biomarker of the present invention and a method of using properties derived from a complex of a substance having an affinity for the biomarker of the present invention and the biomarker of the present invention. Among these, a method of detecting a signal derived from a labeling substance bound to a substance having an affinity for the biomarker of the present invention is preferable.

As a method of detecting a signal derived from a labeling substance bound to a substance having an affinity for the biomarker of the present invention, specifically, for example, a signal derived from a labeling substance bound to a substance having an affinity for the biomarker of the present invention may be detected by a method known per se. For example, the measurement may be performed by a method of the related art such as the immunoassay, for example, the method described in "Enzyme immunoassay" (protein, nucleic acid, enzyme separate volume No. 31, edited by Tsunehiro Kitagawa, Toshio Minamihara, Akio Tsuji, Eiji Ishikawa, pp. 51 to 63, Kyoritsu Shuppan Co., Ltd., 1987) in a case where the labeling substance is an enzyme, and the measurement may be performed by appropriately selecting a measuring device such as a liquid immersion type GM counter, a liquid scintillation counter, a well type scintillation counter, and an HPLC counter and using the selected device according to the kind and the intensity of radiation emitted by a radioactive material using a known method such as RIA in a case where the labeling substance is a radioactive material (see, for example, Medical Chemistry Experiment Course, Volume 8, supervised by Yuichi Yamamura, 1st edition, Nakayama Shoten, 1971). In addition, the measurement may be performed in conformity with a known method such as FIA using a measuring device such as a fluorometer, for example, a method described in "Illustrated Fluorescent Antibody, by Akira Kawao, 1st Edition, Soft Science Corporation, 1983" in a case where the labeling substance is a fluorescent substance, and the measurement may be performed in conformity with a known method using a measuring device such as a photo counter, for example, a method described in "Enzyme immunoassay" (protein, nucleic acid, enzyme separate volume No. 31, edited by Tsunehiro Kitagawa, Toshio Minamihara, Akio Tsuji, Eiji Ishikawa, pp. 252 to 263, Kyoritsu Shuppan Co., Ltd., 1987) in a case where the labeling substance is a luminescent substance. Further, the measurement may be performed by a known method of using a measuring device such as a spectrophotometer in a case where the labeling substance is a substance having absorption in an ultraviolet region, and the measurement may be performed in conformity with a known method of using an electron spin resonance device, for example, a method described in "Enzyme immunoassay" (protein, nucleic acid, enzyme, separate volume No. 31, edited by Tsunehiro Kitagawa, Toshio Minamihara, Akio Tsuji, Eiji Ishikawa, pp. 264 to 271, Kyoritsu Shuppan Co., Ltd., 1987) in a case where the labeling substance has spin properties.

Specific examples of the method of using properties derived from a complex of the substance having an affinity for the biomarker of the present invention and the biomarker of the present invention include a homogeneous immunoassay method such as a surface plasmon resonance method.

It should be noted that a method known per se may be used as a specific method of the method described above.

Hereinafter, the method (A) of using a substance having an affinity for the biomarker of the present invention will be described in more detail.

It should be noted that in the following description, the core protein (or the peptide fragment) in the biomarker of the present invention is referred to as "target core protein (or target peptide fragment)", and the sugar chain in the biomarker of the present invention is referred to as "target sugar chain".

For example, in a case where the biomarker (1) of the present invention is measured, the alpha 1-antitrypsin denotes "target core protein (or target peptide fragment)", and at least one sugar chain represented by the formula 1 or 2 denotes "target sugar chain".

In a case where the biomarker (2) of the present invention is measured, the leucine-rich alpha 2-glycoprotein denotes "target core protein (or target peptide fragment)", and the sugar chain represented by the formula 1 denotes "target sugar chain".

In a case where the biomarker (3) of the present invention is measured, the alpha 1-antichymotrypsin denotes "target core protein (or target peptide fragment)", and at least one sugar chain represented by the formula 1 or 2 denotes "target sugar chain".

In a case where the biomarker (4) of the present invention is measured, the complement component 9 denotes "target core protein (or target peptide fragment)", and the sugar chain represented by the formula 1 denotes "target sugar chain".

Specific examples of the method (A) of using a substance having an affinity for the biomarker of the present invention include a method including the following steps 1 and 2.
(Step 1) A step of separating a target core protein (biomarker of the present invention) to which a target sugar chain is bound from a sample, and
(Step 2) A step of measuring the amount of the target core protein to which the target sugar chain separated in the step 1 is bound

The step 1 may be performed by any method as long as the method enables separation of the target core protein to which the target sugar chain is bound from the sample, and specific examples thereof include a method of separating the target core protein to which the target sugar chain present in the sample is bound from another component. As such a method, specifically, for example, a complex (a substance that specifically binds to a target core protein-a substance that specifically binds to a target sugar chain of the biomarker of the present invention) of a target core protein to which a target sugar chain is bound and a substance having an affinity is formed using two or more substances having an affinity, that is, a substance that specifically binds to a target sugar chain and a substance that specifically binds to a target core protein, and the target core protein to which the target sugar chain is bound may be separated by a method known per se.

In addition, for example, the target core protein (biomarker of the present invention) to which the target sugar chain is bound may be separated from the sample by a method of separating a component that interacts with the target sugar chain of the biomarker of the present invention from another component using an interaction between the substance (for example, a lectin that specifically binds to the sugar chain in the biomarker of the present invention) that specifically binds to the target sugar chain and the biomarker of the present invention and separating the biomarker of the present invention from another component using the substance that specifically binds to the target core protein, for example, a method of separating the biomarker of the present invention from another component using the substance that specifically binds to the target core protein and separating the component that interacts with the target sugar chain of the biomarker of the present invention from another component using the interaction between the substance (for example, a lectin that specifically binds to the sugar chain in the biomarker of the present invention) that specifically binds to the target sugar chain and the biomarker of the present invention. The biomarker of the present invention can be separated by, for example, a lectin electrophoresis method or a lectin column method.

It should be noted that in a case where a substance having an affinity which has been labeled with a labeling substance is used, the separation of the target protein to which the target sugar chain is bound may be paraphrased as separation of a complex of the substance having an affinity which has been labeled with a labeling substance and the biomarker of the present invention. In this case, a complex of the substance having an affinity which has been labeled with a labeling substance and a component other than the biomarker of the present invention and/or a substance having an affinity which has been labeled with a free labeling substance may be separated from each other. Separation from a component that does not contain, as a constituent component, a substance having an affinity which has been labeled with a labeling substance is not necessary.

In addition, the substance having an affinity that specifically binding to the sugar chain and the substance having an affinity that specifically binding to the core protein are as described above, and the specific examples are the same as described above.

The step 2 may be performed by any method as long as the method enables measurement of the target core protein to which the target sugar chain separated in the step 1 is bound, and the specific examples are the same as described above.

In the method, all proteins (including the target protein) in the sample are fragmented into peptides in advance before the step 1 is performed, the target peptide fragment to which the target sugar chain is bound is separated in the step 1, and the target peptide fragment to which the target sugar chain is bound may be measured in the step 2.

Specifically, for example, the fragmentation treatment may be carried out by adding a protease such as trypsin, lysyl endopeptidase, or AspN to the sample before the step 1 is performed, and a complex (a substance that specifically binds to the target peptide fragment-a substance that specifically binds to the target sugar chain of the biomarker of the present invention) of the target peptide fragment to which the target sugar chain is bound and a substance having an affinity is formed using the substance that binds to the target sugar chain in the step 1 and the substance that binds to the target peptide fragment, the complex is separated as described above, and the amount of the complex may be measured.

In addition, in a case where all the proteins are fragmented, the concentration may be performed by a column such as a lectin column after the fragmentation. The concentration may be carried out by a method known per se, and a column such as a commercially available lectin column may be used.

Further, in the method, instead of the step 2, the target sugar chain may be separated from the target protein to which the target sugar chain separated in the step 1 is bound, and only the target sugar chain may be measured.

Specifically, for example, the target sugar chain is separated by treating the target protein to which the target sugar chain separated in the step 1 is bound, with glycanase, hydrazine, or the like, and only the amount of the sugar chain may be measured as described above.

### (B) Method using mass spectrometry method

Specific examples of the method using a mass spectrometry method include methods of using a liquid chromatography mass spectrometer (LC/MS), a capillary electrophoresis mass spectrometer (CE/MS), a gas chromatography mass spectrometer (GC/MS), and a liquid chromatography tandem mass spectrometer (LC/MS/MS).

Specifically, as the method, for example, a component in a sample is separated in a separation portion having a chromatograph such as a liquid chromatograph, and various separated components are ionized in a mass spectrometry portion, and separation may further be made for each mass-to-charge ratio (m/z).

It should be noted that the specific method in the method using the mass spectrometry method may be performed by a method known per se or a method described in WO2014/038524A, WO2017/19588A, or WO2018/034346A.

In the method of using the mass spectrometry, all the proteins may be extracted from the sample in advance, and specifically, for example, the method may be performed by adding a solvent such as acetone, methanol, ethanol, trichloroacetic acid, or a hydrochloric acid aqueous solution to the sample and precipitating all the proteins.

In addition, all the proteins may be fragmented before or after extraction of all the proteins from the sample, and concentration may be performed with a column such as a lectin column after the fragmentation, as necessary. The fragmentation and concentration are as described above.

### Preferable measuring step

In the measuring step according to the present invention, it is preferable to measure (i) at least one sugar chain represented by the formula 1 or 2 which is present in the alpha 1-antitrypsin or (ii) a sugar chain represented by the formula 1 which is present in the leucine-rich alpha 2-glycoprotein, more preferable to measure (i) at least one sugar chain represented by the formula 1 or 2 which is present in the alpha 1-antitrypsin, and still more preferable to measure (iii) at least one sugar chain represented by the formula 1 or 2 which is bound to the amino acid residue (asparagine residue) at position 70 from the N-terminal of the amino acid sequence of the alpha 1-antitrypsin.

In addition, in a case of performing the measurement, a method of using a substance having an affinity for the biomarker of the present invention is preferable, an immunoturbidmetry method such as an ELISA method, an EIA method, an RIA method, an FIA method, a CLEIA method, or a latex coagulating nephelometry method, an immononephelometry method, an immunochromatography method, a western blotting method, an LOCI method, an LBA-EATA method, a capillary electrophoresis method such as a lectin electrophoresis method, a surface plasmon resonance method, and a lectin column method are more preferable, and an ELISA method, an EIA method, an RIA method, an FIA method, a CLEIA method, an LBA-EATA method, and a capillary electrophoresis method such as a lectin electrophoresis method are still more preferable.

A method of bringing a substance having an affinity for the biomarker of the present invention and the biomarker of the present invention into contact with each other, separating the biomarker of the present invention from the substance to form a complex of the substance having an affinity for the biomarker of the present invention and the biomarker of the present invention, and measuring the amount of the complex is more preferable, and a method of bringing a substance having an affinity for the biomarker of the present invention and the biomarker of the present invention into contact with each other, forming a complex of the substance having an affinity for the biomarker of the present invention and the biomarker of the present invention, and detecting a signal derived from the labeling substance bound to the complex is still more preferable.

In addition, a method of separating a component that interacts with the target sugar chain such as the biomarker of the present invention from another component using the interaction between a substance that specifically binds to a target sugar chain (for example, a lectin that specifically binds to a sugar chain in the biomarker of the present invention) and the biomarker of the present invention, separating the biomarker of the present invention from another substance further using a substance that specifically binds to the target core protein, and measuring the amount of the biomarker of the present invention is more preferable, and a method of separating a component that interacts with the target sugar chain such as the biomarker of the present invention from another component using the interaction between a substance that specifically binds to a target sugar chain and the biomarker of the present invention, separating the biomarker of the present invention from another substance using a substance that specifically binds to the target core protein, and detecting a signal derived from the labeling substance bound to a complex of the substance that specifically binds to the target core protein and the biomarker of the present invention is still more preferable.

### Determining step according to present invention

The determining step according to the present invention is a step of determining whether the test animal has colon cancer based on the measurement results obtained in the measuring step according to the present invention.

Specifically, the determining step according to the present invention is performed, for example, by using a value obtained in the measuring step according to the present invention using a sample derived from a test animal (hereinafter, also referred to as a value derived from the test animal) and a preset reference value (cutoff value or the like).

That is, it is possible to determine that "the test animal may have colon cancer or the test animal is highly likely to have colon cancer" in a case where (i) the value derived from the test animal is greater than or equal to the preset reference value (cutoff value or the like) and to determine that "the test animal is unlikely to have colon cancer or the test animal is less likely to have colon cancer" in a case where (ii) the value derived from the test animal is less than the preset reference value (cutoff value or the like).

In addition, as another aspect, it is possible to determine that "the test animal may have colon cancer or the test animal is highly likely to have colon cancer" in a case where (i) the value derived from the test animal is less than or equal to the preset reference value (cutoff value or the like) and to determine that "the test animal is unlikely to have colon cancer or the test animal is less likely to have colon cancer" in a case where (ii) the value derived from the test animal is greater than the preset reference value (cutoff value or the like).

It should be noted that the reference values (cutoff value or the like) are determined based on statistical analysis such as receiver operating characteristics (ROC) curve analysis using the measured value obtained by the measuring step according to the present invention using a sample derived from an animal having colon cancer and the value obtained in the measuring step according to the present invention using a sample derived from a healthy animal (hereinafter, also referred to as the value derived from a healthy animal).

In addition, the sensitivity or/and the specificity of the reference value (cutoff value or the like) is, for example, 60% or greater, preferably 70% or greater, more preferably 80% or greater, and still more preferably 90% or greater.

As another form, it is possible to determine that "the test animal may have colon cancer or the test animal is highly likely to have colon cancer" in a case where the value derived from the test animal is compared to the value derived from a healthy animal and (i) the value derived from the test animal is greater than the value derived from a healthy animal and to determine that "the test animal is unlikely to have colon cancer or the test animal is less likely to have colon cancer" in a case where a significant difference between the value derived from the test animal and (ii)the value derived from a healthy animal is not recognized.

As still another form, it is possible to diagnose the degree of progression and malignancy of colon cancer and to diagnose the prognosis after surgery by comparing the value derived from the test animal at a certain time point and the value derived from the test animal at a different time point in the same test animal and evaluating the presence or absence and/or a degree of increase or decrease of the value. That is, it is possible to determine that the disease state of colon cancer has progressed (or the malignancy of colon cancer has increased) or there is a sign that the disease state of colon cancer has progressed (or there is a sign that the malignancy of colon cancer has increased) in a case where (i) an increase in value is recognized and to determine that the disease state of colon cancer has improved or there is a sign of improvement of the disease state of colon cancer in a case where (ii) a decrease in value is recognized.

It should be noted that in a case where the measurement is performed using a plurality of kinds of the biomarkers of the present invention in the measuring step according to the present invention, the determining step according to the present invention may be performed using the obtained plurality of values as described above, and determination with high accuracy can be made.

### Reagent kit for assisting diagnosis of colon cancer of present invention

A reagent kit for diagnosing colon cancer of the present invention (hereinafter, also referred to as the kit of the present invention) contains a substance having an affinity for the sugar chain selected from the following sugar chains (1) to (4).
(1) At least one sugar chain represented by the formula 1 or 2 which is present in alpha 1-antitrypsin,
(2) a sugar chain represented by the formula 1 which is present in leucine-rich alpha 2-glycoprotein,
(3) at least one sugar chain represented by the formula 1 or 2 which is present in alpha 1-antichymotrypsin, and
(4) a sugar chain represented by the formula 1 which is present in the complement component 9.

The kit of the present invention contains a substance having an affinity for the biomarker of the present invention, that is, the biomarkers (1) to (4) of the present invention.

It should be noted that the biomarker and colon cancer of the present invention in the kit of the present invention are as described in the sections <Biomarker of present invention> and <Assisting method of present invention>, and the specific examples, the preferred examples and the like are also the same as described above.

The substance having the affinity is a substance that specifically binds to a sugar chain or a protein (or a peptide fragment) in the biomarker of the present invention, and examples thereof include lectins and antibodies.

The substance having the affinity is as described in the section of <Assisting method of present invention>, and the specific examples, the preferred examples and the like are also the same as described above.

Further, the kit of the present invention may further contain reagents typically used in this field, for example, reagents for peptide fragmentation such as trypsin, lysyl endopeptidase, and AspN, columns for concentration such as lectin columns, stabilizers such as solvents for protein extraction, buffers, cleaning agents, reaction accelerators, sugars, proteins, salts, and surfactants, preservatives, liquids for diluting samples, immobilized solid phases such as lectin-immobilized solid phases, antibody-immobilized solid phases, and antigen-immobilized solid phases, secondary antibodies or fragments of secondary antibodies labeled with a labeling substance, and reagents for detecting a labeling substance, which do not impair the stability of coexisting reagents and the like. In addition, the concentration and the pH thereof may be in the ranges typically used in this field.

The immobilized solid phases such as the lectin-immobilized solid phases, antibody-immobilized solid phases, and antigen-immobilized solid phases are not limited as long as the immobilized solid phase is obtained by immobilizing the substance (such as a lectin, an antibody, or a fragment of the antibody) having an affinity which specifically binds to a protein (or a peptide fragment) or a sugar chain in the biomarker of the present invention on a material such as magnetic particles such as magnetic silica particles, polystyrene, polycarbonate, polyvinyltoluene, polypropylene, polyethylene, polyvinyl chloride, nylon, polymethacrylate, gelatin, agarose, cellulose, polyethylene terephthalate, glass, or ceramic.

As the material in the immobilized solid phases such as the lectin-immobilized solid phases, the antibody-immobilized solid phases, and the antigen-immobilized solid phases, those produced by a method known per se or commercially available materials may be used. For example, in a case where magnetic particles such as the magnetic silica particles are produced by a method known per se, the magnetic particles can be produced by the method described in WO2012/173002A.

The secondary antibody or the antibody fragment thereof labeled with the labeling substance is an antibody or an antibody fragment thereof that binds to the solid-phased substance having an affinity (such as a lectin, an antibody, or a fragment of the antibody).

It should be noted that the labeling substance and the method of binding the labeling substance in the secondary antibody or the antibody fragment thereof labeled with the labeling substance are as described in the section of <Assisting method of present invention>, and the preferred examples and the specific examples are the same as described above.

The reagent for detecting a labeling substance is a reagent for detecting labeling in the substance having an affinity such as a lectin, an antibody, or a fragment of the antibody labeled with the labeling substance and/or detecting labeling in the labeled secondary antibody or a fragment of the secondary antibody in a case where a substance having an affinity such as a lectin, an antibody, or a fragment of the antibody is labeled with the labeling substance, and examples thereof include substrates for measuring the absorbance such as tetramethylbenzidine and orthophenylenediamine, fluorescent substrates such as hydroxyphenylpropionic acid and hydroxyphenylacetic acid, luminescent substances such as luminol, reagents for measuring the absorbance such as 4-nitrophenyl phosphate, and fluorescent substrates such as 4-methylumbelliferyl phosphate.

Further, the kit of the present invention may include a manual and the like for performing the assisting method of the present invention. The "manual" is an instruction manual, an attached document, a pamphlet (leaflet), or the like for the reagent according to the present invention, in which the features, principles, operating procedures, determination procedures, and the like of the assisting method of the present invention are substantially described in text and/or in figures and tables. Specific examples thereof include a manual in which (i) the principles, the operating procedures, and the like of the measuring step according to the present invention are described and a manual in which (ii) the principles, the operating procedures, and the like of the measuring step and the determining step according to the present invention are described.

According to the kit of the present invention, the assisting method of the present invention can be easily performed in a short time with high accuracy.

### Device for assisting diagnosis of colon cancer according to present invention

A device for assisting the diagnosis of colon cancer according to the present invention (hereinafter, also referred to as an assisting device according to the present invention) includes at least a measuring unit (1). Further, the device may further include a determining unit (2), an output unit (3), and an input unit (4).

The measuring unit (1) in the assisting device according to the present invention is configured to measure the amount of the sugar chain selected from the biomarkers (1) to (4) of the present invention in the sample. Specific examples thereof include measuring devices such as various mass spectrometers used in the measuring step according to the present invention and devices used for a method in conformity with an immunoassay method.

It should be noted that the measuring unit (1) may be configured to calculate the amounts of the biomarkers (1) to (4) of the present invention based on the measured values which have been measured.

The determining unit (2) in the assisting device according to the present invention is configured to determine whether the test animal has colon cancer based on the results obtained by the measuring unit (1).

The output unit (3) in the assisting device according to the present invention is configured to output the result obtained by the measuring unit (1) and/or the result obtained by the determining unit (2).

The input unit (4) in the assisting device according to the present invention is configured to send a signal to the measuring unit (1) for operating the measuring unit (1) in response to the operation of an operator.

It should be noted that the measurement, the determination, and the like performed by the measuring unit (1) and the determining unit (2) of the assisting device according to the present invention are as described in the section of <Assisting method of present invention>, and the preferred examples, the specific examples, and the like are also the same as described above.

According to the assisting device of the present invention, the assisting method of the present invention can be easily performed in a short time with high accuracy.

### Method of treating colon cancer according to present invention

A method of treating colon cancer according to the present invention (hereinafter, also referred to as the treatment method according to the present invention) is performed by measuring the amount of a sugar chain selected from the biomarkers (1) to (4) of the present invention in a sample derived from a test animal, determining whether the test animal has colon cancer based on the obtained measurement results, and performing an appropriate treatment on a patient that is likely to have colon cancer or highly likely to have colon cancer based on the determination results.

The sample, the marker, the measurement, the determination, and the like in the treatment method according to the present invention are as described in the section of <Assisting method of present invention>, and the preferred examples, the specific examples, and the like are also the same as described above.

Specific examples of the appropriate treatment in the treatment method according to the present invention include surgical therapy such as endoscopic treatment, open surgery, or laparoscopic surgery, radiation therapy such as chemoradiation therapy, and drug therapy performed by administering a drug such as OXALIPLATIN (generic name), IRINOTECAN (generic name), FLUOROURACIL (generic name), BEVACIZUMAB (generic name), RAMUCIRUMAB (generic name), AFLIBERCEPT (generic name), CETUXIMAB (generic name), or PANITUMUMAB (generic name).

Hereinafter, the present invention will be described in detail based on the following examples, but the present invention is not limited to such examples.

### Example

### Example 1. Sorting out of colon cancer marker

### (1) Specimen (sample)

Serums (18 specimens) derived from colon cancer patients and serums (20 specimens) derived from healthy subjects provided by Yokohama City University Center Hospital and Advanced Medical Research Center, Yokohama City Biobank were used as specimens (samples).

### (2) Preparation of glycopeptide

A digestive buffer consisting of 100 mM Tris-HCl (pH of 9.0) containing 12 mM of sodium deoxycholate (SDC) and 12 mM of N-lauroylsarcosine (LS) was prepared, and 90 µL of the digestive buffer and 10 µL of the serum derived from a colon cancer patient and the serum derived from a healthy subject described in the section of (1) were mixed with each other. Next, the protein was modified by adding a mercaptoethanol solution thereto such that the final concentration reached 10 mM and incubating the mixture at 95°C for 5 minutes. Next, cysteine was alkylated by adding an acrylamide (AA) solution thereto such that the final concentration reached 20 mM and incubating the mixture in a dark place at 25°C for 30 minutes. Next, the protein was digested by incubating the resultant using 5 µg of Lys-C at 37°C for 1 hour. Next, the protein was further digested by diluting the solution with 400 µL of 50 mM Tris-HCl (pH 8.0) containing 1.5 mM CaCl₂ and incubating the resulting solution using 5 µg of trypsin at 37°C for 12 hours. Thereafter, 500 µL of ethyl acetate and 10 µL of formic acid were respectively added thereto, and the mixture was vortexed for 30 seconds and centrifuged at 14000 × g for 5 minutes. Thereafter, the upper layer (ethyl acetate layer) containing SDC and LC was removed and approximately 30 µL of the solution was evaporated with a centrifugal evaporator, thereby preparing glycopeptides.

### (3) Concentration of glycopeptides

Each glycopeptide of 38 specimens (colon cancer patient: 18 specimens, healthy subject: 20 specimens) prepared in the section of (2) was concentrated by the following procedure.

That is, first, 300 µL of a cellulose microcrystalline resin was washed with 10 mL of 0.1% trifluoroacetic acid (TFA)/90% acetonitrile (ACN) in a 15 mL centrifugal tube. Thereafter, 300 µL of the washed cellulose microcrystalline resin, 10 ml of 0.1% TFA/90% ACN, and 8 µL of each glycopeptide prepared in the section of (2) were mixed and incubated at 1 rpm for 6 minutes. Thereafter, the cellulose microcrystalline resin on which each glycopeptide was adsorbed was washed 4 times with 10 mL of 0.1% TFA/80% ACN. Next, each glycopeptide was washed twice with 600 µL of 30% ACN, and each glycopeptide was eluted from the cellulose microcrystalline resin to concentrate each glycopeptide. It should be noted that the solution was centrifuged at 14000 × g in order to remove fine cellulose particles after the concentration, evaporated by a centrifugal evaporator, and redissolved in 50 µL of 0.1% formic acid/3% ACN.

### (4) Preparation of glyco-free peptide

For identification of the sugar chain modification site, 25 µL of the concentrated glycopeptide sample prepared in the section of (3) was subjected to PNGase F treatment. That is, each of the samples was neutralized by adding 20 µL of 1M Tris-HCl (pH 8.0) and mixed with 1 unit of PNGase F. Next, the mixture was incubated at 37°C for 1 hour and loaded on a desalting column washed with 5 µL ACN and 0.1% formic acid in advance. Glyco-free peptides were prepared by washing the mixture twice with 5 µL of 0.1% formic acid and eluting the mixture with 5 µL of 50% ACN. It should be noted that the mixture was diluted with 20 µL of 0.1% formic acid after the preparation and conserved at -30°C until use.

### (5) Analysis by nano liquid chromatography mass spectrometry (nanoLC/MS/MS)

Each concentrated glycopeptide prepared in the section of (3) was analyzed (separated/analyzed) by an Orbitrap mass spectrometer Q-Exactive (manufactured by Thermo Fisher Scientific).

Specifically, 1.6 µL of each glycopeptide was injected into nanoLC, EASY-nLC1000 (manufactured by Thermo Fisher Scientific) connected to Q-Exactive in a nanobore trap column at 200 bar and washed with 20 µL of 0.1% formic acid. Next, the amount of glycopeptide was measured by eluting the mixture with a binary linear gradient of 0.1% formic acid (solvent A) and 0.1% formic acid/ACN at a flow rate of 300 nL/min (0 to 150 minutes: 0% solvent B to 35% solvent B, 150 to 155 minutes: 100% solvent B).

It should be noted that the nanobore trap column and the analytical column were initialized with 12 µL and 5 µL of 0.1% FA before each analysis. Full MS was monitored from m/z 350 to m/z 2000 with Resolution 70000, and product ion scan was acquired by Data dependent acquisition. The ionization parameters and Q-Exactive Data dependent acquisition parameters in the measurement are as follows.

### Ionization parameters

Spray voltage: 1800 V
Capillary temperature: 250°C
S-lens RF level: 50 (no unit)

It should be noted that auxiliary gas and sheath gas were not used. In addition, the order of measurement of serum samples derived from healthy subjects and colon cancer patients was randomized to avoid influence of confounding factors.

### Data dependent acquisition parameter of Q-Exactive

Resolution: 17500
AGC target: 1000000
Maximum injection time: 350 ms
Isolation width: 3.0m/z
: stepped normalized collision energy: 25 and 35

In addition, 1.6 µL of each glyco-free peptide prepared in the section of (4) was analyzed (separated/analyzed) under the same conditions as described above. Thereafter, the sugar chain binding sites in the glyco-free peptide were identified using Proteome Discoverer 1.4 (manufactured by Thermo Fisher Scientific). Further, the structures of the glycopeptides were confirmed based on the identification information.

### (6) Results

As described in the sections of (1) to (5), glycopeptides A to M derived from the alpha 1-antitrypsin, the leucine-rich alpha 2-glycoprotein, the alpha 1-antichymotrypsin, and the complement component 9 were identified as listed in Table 7.

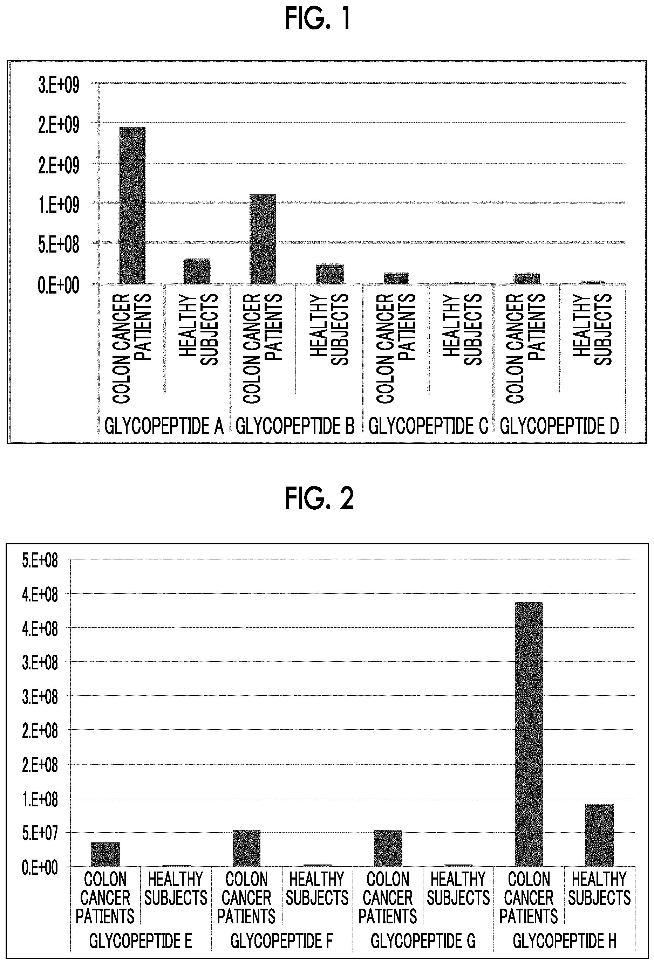

In addition, the average value of the peak surface area values of the glycopeptides A to M derived from colon cancer patients (18 specimens) and the average value thereof derived from healthy subjects (20 specimens) are listed in Table 8 and Figs. 1 to 4.

Table 7

Table 8

As listed in Tables 7 and 8 and shown in Fig. 1, it was found that the expression levels of (i) glycopeptide A having a sugar chain represented by the formula 1" (average peak surface area value of colon cancer patients: 1939358186.22222, average peak surface area value of healthy subjects: 306335029.7), (ii) glycopeptide B having a sugar chain represented by the formula 1‴ (average peak surface area value of colon cancer patients: 1099675503.77778, average peak surface area value of healthy subjects: 245395191.4), (iii) glycopeptide C having a sugar chain represented by the formula 2" (average peak surface area value of colon cancer patients: 132975907.444444, average peak surface area value of healthy subjects: 14027932.45), and (iv) glycopeptide D having a sugar chain represented by the formula 2‴ (average peak surface area value of colon cancer patients: 138263873.055556, average peak surface area value of healthy subjects: 25985005.75), derived from alpha 1-antitrypsin, were significantly increased in the colon cancer patients as compared with the healthy subjects.

In addition, it was confirmed that the sugar chain represented by the formula 1" in the glycopeptide A, the sugar chain represented by the formula 1‴ in the glycopeptide B, the sugar chain represented by the formula 2" in the glycopeptide C, and the sugar chain represented by the formula 2‴ in the glycopeptide D were bound to the asparagine residue at position 70 from the N-terminal of the amino acid sequence of alpha 1-antitrypsin.

As listed in Tables 7 and 8 and shown in Fig. 2, it was found that the expression levels of (i) glycopeptide E having a sugar chain represented by the formula 1" (average peak surface area value of colon cancer patients: 36204545.5555556, average peak surface area value of healthy subjects: 2037297.6), (ii) glycopeptide F having a sugar chain represented by the formula 1ʺʺ (average peak surface area value of colon cancer patients: 53995609.7777778, average peak surface area value of healthy subjects: 3415333.55), (iii) glycopeptide G having a sugar chain represented by the formula 1ʺʺ (average peak surface area value of colon cancer patients: 53995609.7777778, average peak surface area value of healthy subjects: 3415333.55), and (iv) glycopeptide H having a sugar chain represented by the formula 1‴ (average peak surface area value of colon cancer patients: 387390364.555556, average peak surface area value of healthy subjects: 92357205.6), derived from leucine-rich alpha 2-glycoprotein, were significantly increased in the colon cancer patients as compared with the healthy subjects.

In addition, it was confirmed that the sugar chain represented by the formula 1" in the glycopeptide E and the sugar chain represented by the formula 1ʺʺ in the glycopeptide F are bound to the asparagine residue at position 79 from the N-terminal of the amino acid sequence of the leucine-rich alpha 2-glycoprotein. It was confirmed that the sugar chain represented by the formula 1ʺʺ in the glycopeptide G was bound to the asparagine residue at position 186 from the N-terminal of the amino acid sequence of the leucine-rich alpha 2-glycoprotein. It was confirmed that the sugar chain represented by the formula 1‴ in the glycopeptide H was bound to the asparagine residue at position 269 from the N-terminal of the amino acid sequence of the leucine-rich alpha 2-glycoprotein.

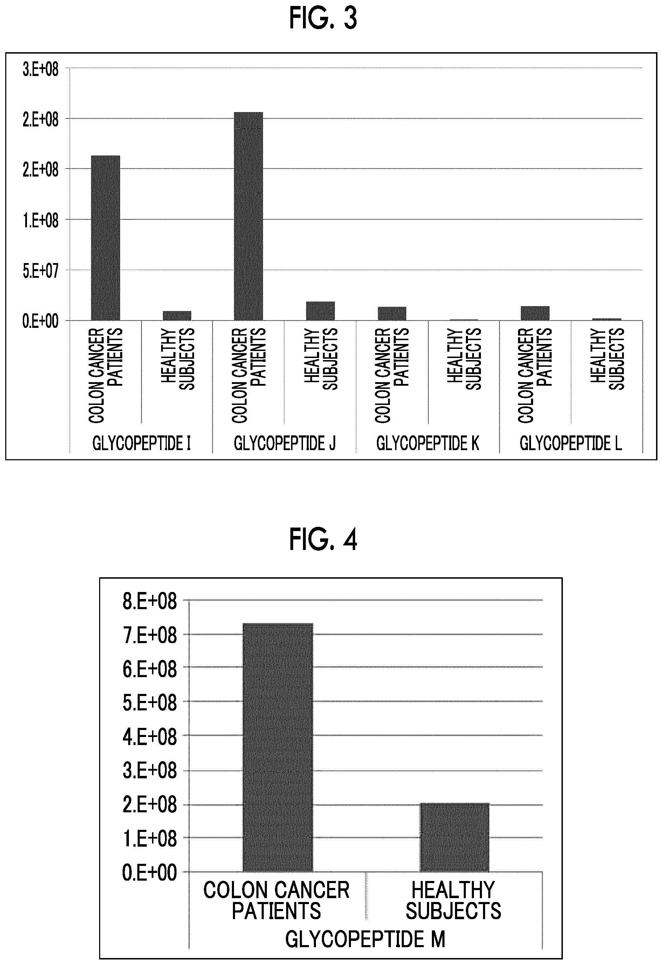

As listed in Tables 7 and 8 and shown in Fig. 3, it was found that the expression levels of (i) glycopeptide I having a sugar chain represented by the formula 2" (average peak surface area value of colon cancer patients: 162903669.944444, average peak surface area value of healthy subjects: 8899040.75), (ii) glycopeptide J having a sugar chain represented by the formula 2‴ (average peak surface area value of colon cancer patients: 206514550.444444, average peak surface area value of healthy subjects: 18952737.35), (iii) glycopeptide K having a sugar chain represented by the formula 1" (average peak surface area value of colon cancer patients: 13321110.3333333, average peak surface area value of healthy subjects: 913077), and (iv) glycopeptide L having a sugar chain represented by the formula 1‴ (average peak surface area value of colon cancer patients: 14098497.3333333, average peak surface area value of healthy subjects: 2290770.7), derived from alpha 1-antichymotrypsin, were significantly increased in the colon cancer patients as compared with the healthy subjects.

In addition, it was confirmed that the sugar chain represented by the formula 2" in the glycopeptide I, the sugar chain represented by the formula 2‴ in the glycopeptide J, the sugar chain represented by the formula 1" in the glycopeptide K, and the sugar chain represented by the formula 1‴ in the glycopeptide L were bound to the asparagine residue at position 93 from the N-terminal of the amino acid sequence of the alpha 1-antichymotrypsin.

As listed in Tables 7 and 8 and shown in Fig. 1, it was found that the expression levels of (i) glycopeptide M having a sugar chain represented by the formula 1‴ (average peak surface area value of colon cancer patients: 733351267.666667, average peak surface area value of healthy subjects: 202557121.85), derived from complement component 9, were significantly increased in the colon cancer patients as compared with the healthy subjects.

Further, area under the curves (AUC, curve area value) for the expression levels of the glycopeptides A to M were calculated by receiver operating characteristic (ROC) curve analysis.

The results are listed in Table 9. It should be noted that the determination ability increases as the AUC value is close to 1.

Table 9

As listed in Table 9, all the glycopeptides A to M showed satisfactory AUC values.

As described above, it was found that determination whether a subject has colon cancer can be made by using the glycopeptides A to M as biomarkers for colon cancer.

### [Sequence list]

(2139PCT)0002.txt

## Claims

1. A biomarker for assisting diagnosis of colon cancer, comprising: a sugar chain selected from the following sugar chains (1) to (4),
(1) at least one sugar chain represented by the following formula 1 or 2 which is present in alpha 1-antitrypsin;
(2) a sugar chain represented by the following formula 1 which is present in leucine-rich alpha 2-glycoprotein;
(3) at least one sugar chain represented by the following formula 1 or 2 which is present in alpha 1-antichymotrypsin; and
(4) a sugar chain represented by the following formula 1 which is present in a complement component 9, 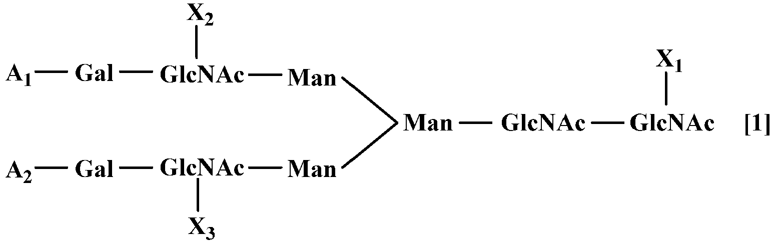
in the formula 1, X₁, X₂, and X₃ each independently represent Fuc or H, and A₁ and A₂ each independently represent Neu5Ac, Neu5Ac-Neu5Ac, or H 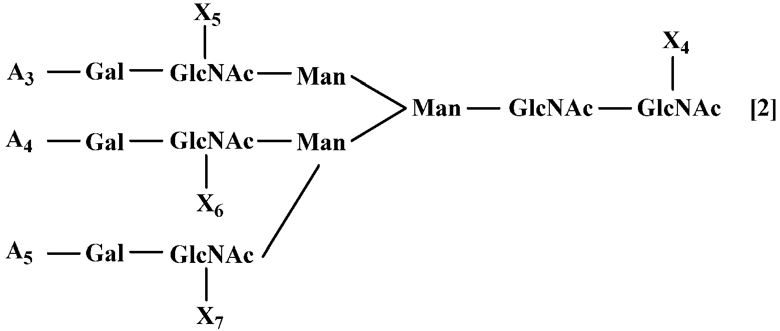
in the formula 2, X₄, X₅, X₆, and X₇ each independently represent Fuc or H, and A₃, A₄, and A₅ each independently represent Neu5Ac, Neu5Ac-Neu5Ac, or H.

2. The biomarker according to claim 1,
wherein the sugar chain is a sugar chain represented by any one of the following sugar chains (1-1) to (4-1),
(1-1) at least one sugar chain represented by the formula 1 or 2 which is bound to an asparagine residue at position 70 from a N-terminal of an amino acid sequence of the alpha 1-antitrypsin;
(2-1) a sugar chain represented by the formula 1 which is bound to an asparagine residue at position 79, an asparagine residue at position 186, or an asparagine residue at position 269 from a N-terminal of an amino acid sequence of the leucine-rich alpha 2-glycoprotein;
(3-1) at least one sugar chain represented by the formula 1 or 2 which is bound to an asparagine residue at position 93 from a N-terminal of an amino acid sequence of the alpha 1-antichymotrypsin; and
(4-1) a sugar chain represented by the formula 1 which is bound to an asparagine residue at position 415 from a N-terminal of an amino acid sequence of the complement component 9.

3. The biomarker according to claim 1,
wherein the sugar chain is at least one sugar chain represented by the formula 1 or 2 which is bound to an asparagine residue at position 70 from a N-terminal of an amino acid sequence of the alpha 1-antitrypsin.

4. A method of assisting diagnosis of colon cancer, comprising: measuring a sugar chain selected from the following sugar chains (1) to (4) in a sample derived from a test animal; and determining whether the test animal has colon cancer based on an obtained measurement result,
(1) at least one sugar chain represented by the following formula 1 or 2 which is present in alpha 1-antitrypsin;
(2) a sugar chain represented by the following formula 1 which is present in leucine-rich alpha 2-glycoprotein;
(3) at least one sugar chain represented by the following formula 1 or 2 which is present in alpha 1-antichymotrypsin; and
(4) a sugar chain represented by the following formula 1 which is present in a complement component 9, 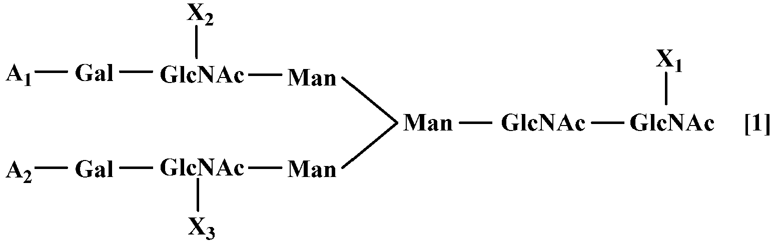
in the formula 1, X₁, X₂, and X₃ each independently represent Fuc or H, and A₁ and A₂ each independently represent Neu5Ac, Neu5Ac-Neu5Ac, or H 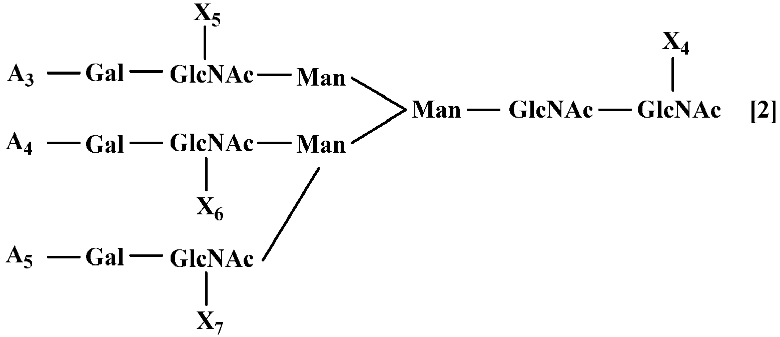
in the formula 2, X₄, X₅, X₆, and X₇ each independently represent Fuc or H, and A₃, A₄, and A₅ each independently represent Neu5Ac, Neu5Ac-Neu5Ac, or H.

5. The method according to claim 4,
wherein the sugar chain is a sugar chain represented by any one of the following sugar chains (1-1) to (4-1),
(1-1) at least one sugar chain represented by the formula 1 or 2 which is bound to an asparagine residue at position 70 from a N-terminal of an amino acid sequence of the alpha 1-antitrypsin;
(2-1) a sugar chain represented by the formula 1 which is bound to an asparagine residue at position 79, an asparagine residue at position 186, or an asparagine residue at position 269 from a N-terminal of an amino acid sequence of the leucine-rich alpha 2-glycoprotein;
(3-1) at least one sugar chain represented by the formula 1 or 2 which is bound to an asparagine residue at position 93 from a N-terminal of an amino acid sequence of the alpha 1-antichymotrypsin; and
(4-1) a sugar chain represented by the formula 1 which is bound to an asparagine residue at position 415 from a N-terminal of an amino acid sequence of the complement component 9.

6. The method according to claim 4,
wherein the sugar chain is at least one sugar chain represented by the formula 1 or 2 which is bound to an asparagine residue at position 70 from a N-terminal of an amino acid sequence of the alpha 1-antitrypsin.

7. The method according to claim 4,
wherein the sample is whole blood, serum, or plasma.

8. A reagent kit for assisting diagnosis of colon cancer, comprising: a substance having an affinity for a sugar chain selected from the following sugar chains (1) to (4),
(1) at least one sugar chain represented by the following formula 1 or 2 which is present in alpha 1-antitrypsin;
(2) a sugar chain represented by the following formula 1 which is present in leucine-rich alpha 2-glycoprotein;
(3) at least one sugar chain represented by the following formula 1 or 2 which is present in alpha 1-antichymotrypsin; and
(4) a sugar chain represented by the following formula 1 which is present in a complement component 9, 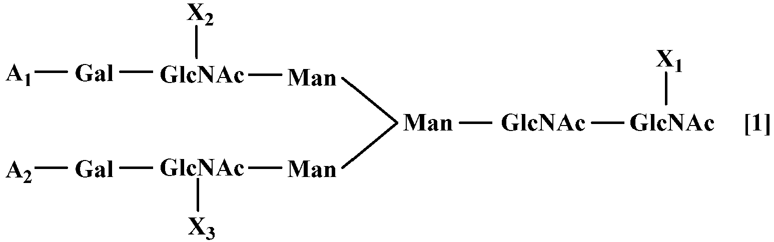
in the formula 1, X₁, X₂, and X₃ each independently represent Fuc or H, and A₁ and A₂ each independently represent Neu5Ac, Neu5Ac-Neu5Ac, or H 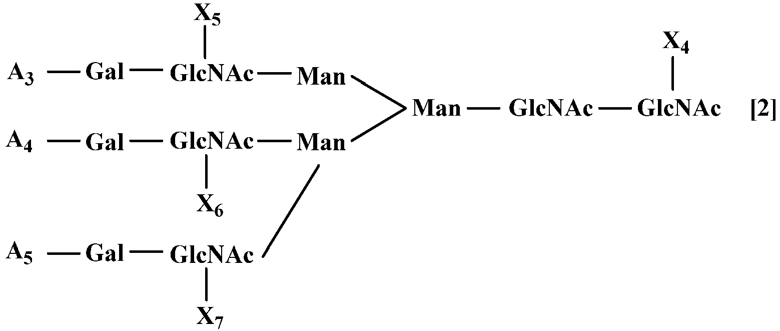
in the formula 2, X₄, X₅, X₆, and X₇ each independently represent Fuc or H, and A₃, A₄, and A₅ each independently represent Neu5Ac, Neu5Ac-Neu5Ac, or H.

9. The reagent kit according to claim 8,
wherein the sugar chain is a sugar chain represented by any one of the following sugar chains (1-1) to (4-1),
(1-1) at least one sugar chain represented by the formula 1 or 2 which is bound to an asparagine residue at position 70 from a N-terminal of an amino acid sequence of the alpha 1-antitrypsin;
(2-1) a sugar chain represented by the formula 1 which is bound to an asparagine residue at position 79, an asparagine residue at position 186, or an asparagine residue at position 269 from a N-terminal of an amino acid sequence of the leucine-rich alpha 2-glycoprotein;
(3-1) at least one sugar chain represented by the formula 1 or 2 which is bound to an asparagine residue at position 93 from a N-terminal of an amino acid sequence of the alpha 1-antichymotrypsin; and
(4-1) a sugar chain represented by the formula 1 which is bound to an asparagine residue at position 415 from a N-terminal of an amino acid sequence of the complement component 9.
